# EUROPÄISCHE PATENTSCHRIFT

(11) **EP 3 282 016 B1**
(45) Veröffentlichungstag und Bekanntmachung des Hinweises auf die Patenterteilung: **20.08.2025**
(21) Anmeldenummer: 16183533.5
(22) Anmeldetag: 10.08.2016
(51) Int. Cl.: C12N 15/82, C07K 14/415, C12Q 1/68

(54) **RESISTENZGEN GEGEN WURZELBÄRTIGKEIT**
RESISTANCE AGAINST RHIZOMANIA
GENES DE RESISTANCE CONTRE LA RHIZOMANIE

(43) Veröffentlichungstag der Anmeldung: 14.02.2018
(73) Patentinhaber: KWS SAAT SE & Co. KGaA, 37574 Einbeck (DE)
(72) Erfinder: Törjék, Ottó, 37574 Einbeck (DE); Borchardt, Dietrich, 37574 Einbeck (DE); Mechelke, Wolfgang, 37574 Einbeck (DE); Lein, Jens Christoph, 37085 Göttingen (DE); Habekost, Sandra, 37586 Dassel (DE)
(74) Vertreter: Michalski Hüttermann & Partner Patentanwälte mbB

(56) Entgegenhaltungen:
- WO-A1-2014/202044
- DATABASE EMBL [online] 8 July 2015 (2015-07-08), "Beta vulgaris subsp. vulgaris hypothetical protein", XP002765844, retrieved from EBI accession no. KMT15745 Database accession no. KMT15745
- SCHOLTEN O E ET AL: "Inheritance of resistance to beet necrotic yellow vein virus in Beta vulgaris conferred by a second gene for resistance", THEORETICAL AND APPLIED GENETICS ; INTERNATIONAL JOURNAL OF PLANT BREEDING RESEARCH, SPRINGER, BERLIN, DE, vol. 99, no. 3 - 4, 1 August 1999 (1999-08-01), pages 740 - 746, XP035065867, ISSN: 1432-2242, DOI: 10.1007/S001220051292
- AMIRI R ET AL: "A new RAPD marker for beet necrotic yellow vein virus resistance gene in Beta vulgaris", BIOLOGIA PLANTARUM, KLUWER ACADEMIC PUBLISHERS, DO, vol. 53, no. 1, 21 March 2009 (2009-03-21), pages 112 - 119, XP019670809, ISSN: 1573-8264
- GRIMMER M K ET AL: "An anchored linkage map for sugar beet based on AFLP, SNP and RAPD markers and QTL mapping of a new source of resistance to Beet necrotic yellow vein virus", THEORETICAL AND APPLIED GENETICS ; INTERNATIONAL JOURNAL OF PLANT BREEDING RESEARCH, SPRINGER, BERLIN, DE, vol. 114, no. 7, 9 February 2007 (2007-02-09), pages 1151 - 1160, XP019510491, ISSN: 1432-2242, DOI: 10.1007/S00122-007-0507-3

## Beschreibung

### GEBIET DER ERFINDUNG

Die vorliegende Erfindung betrifft ein Nukleinsäuremolekül, welches ein Polypeptid kodiert, das in der Lage ist, eine Resistenz gegenüber einem Pathogen, insbesondere gegenüber dem "Beet Necrotic Yellow Vein Virus" (BNYVV) in einer Pflanze, insbesondere der Gattung Beta, in welcher das Polypeptid exprimiert wird, zu vermitteln, wie im Folgenden näher erläutert. Die Erfindung betrifft weiterhin eine transgene Pflanzenzelle, eine transgene Pflanze, eine BNYVV-resistente Pflanze oder Teile davon, oder einen Samen einer Pflanze, welche das Nukleinsäuremolekül umfasst, in welchem durch Einbringung einer oder mehrerer Mutationen die Resistenz hervorgerufen wird. Verfahren zur Herstellung einer BNYVV-resistenten Pflanze, zur Identifikation einer BNYVV-resistenten Pflanze der Gattung Beta, und zur Kontrolle des Befalls von Pflanzen der Gattung Beta mit BNYVV werden ebenso von der vorliegenden Erfindung umfasst.

### HINTERGRUND DER ERFINDUNG

Die Wurzelbärtigkeit (Rizomania) ist die wirtschaftlich bedeutendste Zuckerrübenkrankheit weltweit, die Ertragsverluste von 50 % und mehr verursachen kann. Die Krankheit, die auch "Wurzelbärtigkeit" genannt wird, wird durch das "Beet Necrotic Yellow Vein Virus" (BNYVV) hervorgerufen und durch den bodenbürtigen Protozoen *Polymyxa betae* übertragen. Eine BNYVV-Infektion äußert sich in einer gesteigert Proliferation der dünnen Wurzeln und Seitenwurzeln und in der Ausbildung eines stark verkleinerten Wurzelkörpers mit reduzierten Zuckergehalt. Infizierte Pflanzen zeigen eine verminderte Wasseraufnahme und sind somit empfindlicher gegenüber Trockenstress. Wenn sich die Infektion auf die Gesamtpflanze ausweitet, kommt es zur Gelbfärbung der Blattvenen, zu nekrotischen Läsionen und gelben Flecken auf den Blättern. Da eine kurative Bekämpfung der Krankheit wie bei anderen viralen Krankheiten nicht möglich ist, kann ein Schaden nur über den Anbau von resistenten Sorten verhindert werden. Die Entwicklung von Genotypen mit einer genetischen Resistenz gegen Rizomania ist somit entscheidend für die Züchtung von Zuckerrübe.

Die ersten Züchtungsprogramme für Rizomaniaresistenz begannen bereits 1981 in Italien als man erkannte, dass in kommerziellen Keimplasmen eine zufriedenstellende genetische Variabilität für Rizomaniaresistenz vorhanden ist. Pflanzen und Genotypen, welche abgemilderte Krankheitssymptome und nahezu normales Wurzelgewicht und Zuckergehalt aufwiesen, wurden selektiert. Dabei gelang es eine multigenische Resistenzquelle namens "Alba type" aus Kreuzungen mit *Beta vulgaris* L. subsp. *maritima* (L.) zu identifizieren.

Im Jahre 1982 wurde ein weiterer Resistenztyp entwickelt, der verbesserten Schutz gegenüber Rizomania zeigte. Dieser tauchte zwei Jahre später in der Sorte Rizor auf und wurde als monogenische, dominante Resistenz klassifiziert. Eine weitere monogenische, dominante Resistenz wurde im Jahre 1983 bei Sorten-Versuchen in Kalifornien bei dem Züchtungsunternehmen "Holly Sugar Company" aufgefunden, welche bereits drei Jahre später auch in Europa eingeführt wurde (Lewellen *et al.* 1987). Diese als RZ-1 bekannte Resistenz (auch als "Holly" bezeichnet) entwickelte sich zu der meistverwendeten Quelle und löste schnell Rizor ab. Wenige Jahre später wurde eine neue Quelle für Rizomaniaresistenz in der *Beta maritima* Akzession WB42 aus Dänemark gefunden. Sie zeigte eine zu RZ-1 unterschiedliche Kartierungsposition auf Chromosom III und vermittelte zudem ein erhöhtes Resistenzlevel verglichen mit RZ-1. Dieses neue Majorgen wurde als RZ-2 bezeichnet. Mittlerweile ist weiterhin auch RZ-3 aus der *Beta maritima* Akzession WB41 bekannt, bei welchem es sich wahrscheinlich um eine allelische Variante des RZ-2 handelt.

Bis heute ist RZ-3 das einzige Rizomaniaresistenzgen, dessen funktioneller Hintergrund, d. h. die genetische Struktur, aufgeklärt wurde; siehe die Deutsche Patentanmeldung DE 10 2013 010 026 A2), wodurch die züchterische Nutzung des Gens deutlich verbessert werden konnte. Somit besteht der anhaltende Wunsch, die bereits bekannten, sowie auch neue Resistenzquellen genetisch besser zu beschreiben, um damit die Entwicklung molekularer Marker voranzutreiben. Somit könnten Zuckerrübe-Elitelinien durch Eliminierung des "Linkage Drag" weiter optimiert werden aber auch neue Resistenzquellen identifiziert werden.

Für eine nachhaltige Züchtung gegen Wurzelbärtigkeit, die der Gefahr von Resistenz-brechenden BNYVV-Isolaten entgegen wirken soll, ist es notwendig, ständig neue Resistenzgene zu identifizieren und diese in die Genpools der Kulturpflanzen wie Zuckerrüben zu integrieren. Diese Aufgabe wird erfindungsgemäß durch die in den Ansprüchen und in der Beschreibung gekennzeichneten Ausführungsformen gelöst.

### ZUSAMMENFASSUNG DER ERFINDUNG

Die vorliegende Erfindung betrifft ein Nukleinsäuremolekül, das in der Lage ist, eine Resistenz gegenüber einem Pathogen, insbesondere gegenüber dem "Beet Necrotic Yellow Vein Virus" (BNYVV) in einer Pflanze, insbesondere der Gattung Beta zu vermitteln, in welcher ein Polypeptid, welches durch das Nukleinsäuremolekül kodiert wird, exprimiert wird. Die Erfindung betrifft weiterhin eine transgene Pflanzenzelle, eine transgene Pflanze, eine BNYVV-resistente Pflanze oder Teile davon, oder einen Samen einer Pflanze, welche das Nukleinsäuremolekül umfasst, in welchem die Resistenz durch Einführung einer oder mehrerer Mutationen vermittelt wird. Verfahren zur Herstellung einer BNYVV-resistenten Pflanze, zur Identifikation einer BNYVV-resistenten Pflanze der Gattung Beta, und zur Kontrolle des Befalls von Pflanzen der Gattung Beta mit BNYVV werden ebenso von der vorliegenden Erfindung umfasst.

Die vorliegende Erfindung wird definiert durch die Merkmale der unabhängigen Ansprüche.

Gemäß einem ersten Aspekt betrifft die vorliegende Erfindung ein Nukleinsäuremolekül, welches ein Polypeptid kodiert, das in der Lage ist, eine Resistenz gegenüber einem Pathogen in einer Pflanze, in welcher das Polypeptid exprimiert wird, zu vermitteln, dadurch gekennzeichnet, dass das Nukleinsäuremolekül eine Nukleotidsequenz umfasst, welche ausgewählt ist aus
(a) einer Nukleotidsequenz, die ein Polypeptid mit einer Aminosäuresequenz, die zu mindestens 80 % identisch mit der SEQ ID NO: 2 ist, kodiert, wobei das Nukleinsäuremolekül eine oder mehrere Mutationen aufweist, welche dazu führen, dass das Polypeptid, das durch das Nukleinsäuremolekül kodiert wird, an derjenigen Position, welche in der Referenz-Aminosäuresequenz SEQ ID NO: 2 der Position 307 entspricht, eine Aminosäure aufweist, welche sich von Lysin (K) unterscheidet, und/oder an derjenigen Position, welche in der Referenz-Aminosäuresequenz SEQ ID NO: 2 der Position 437 entspricht, eine Aminosäure aufweist, welche sich von Glutamin (Q) unterscheidet; und
(b) einer Nukleotidsequenz, die mit der komplementären Sequenz zu SEQ ID NO: 1 unter stringenten Bedingungen hybridisiert, wobei die Nukleotidsequenz an denjenigen Stellen, die den Stellen in der Referenz-Nukleotidsequenz SEQ ID NO: 1 entsprechen, eine oder mehrere der folgenden Nukleotidsubstitutionen aufweist: C statt A an Position 919; G statt A an Position 1310; A statt G an Position 1697; A statt C an Position 2191; und/oder T statt C an Position 2491;
vorzugsweise wobei das Pathogen das Beet necrotic yellow vein virus (BNYVV) und die Pflanze eine Pflanze der Gattung Beta, vorzugsweise Zuckerrübe (Beta vulgaris L.), ist.

Das besagte Nukleinsäuremolekül kann dadurch gekennzeichnet sein, dass das kodierte Polypeptid weiterhin (i) an derjenigen Position, welche in der Referenz-Aminosäuresequenz SEQ ID NO: 2 der Position 566 entspricht, eine Aminosäure aufweist, welche sich von Arginin (R) unterscheidet, und/oder (ii) an derjenigen Position, welche in der Referenz-Aminosäuresequenz SEQ ID NO: 2 der Position 731 entspricht, eine Aminosäure aufweist, welche sich von Glutamin (Q) unterscheidet, und/oder (iii) an derjenigen Position, welche in der Referenz-Aminosäuresequenz SEQ ID NO: 2 der Position 831 entspricht, eine Aminosäure aufweist, welche sich von Prolin (P) unterscheidet.

Das besagte Nukleinsäuremolekül kann dadurch gekennzeichnet sein, dass das kodierte Polypeptid (i) ein Glutamin (Q) an derjenigen Position aufweist, welche in der Referenz-Aminosäuresequenz SEQ ID NO: 2 der Position 307 entspricht, und/oder (ii) ein Arginin (R) an derjenigen Position aufweist, welche in der Referenz-Aminosäuresequenz SEQ ID NO: 2 der Position 437 entspricht, und/oder (iii) ein Histidin (H) an derjenigen Position aufweist, welche in der Referenz-Aminosäuresequenz SEQ ID NO: 2 der Position 566 entspricht, und/oder (iv) ein Lysin (K) an derjenigen Position aufweist, welche in der Referenz-Aminosäuresequenz SEQ ID NO: 2 der Position 731 entspricht, und/oder (v) ein Serin (S) an derjenigen Position aufweist, welche in der Referenz-Aminosäuresequenz SEQ ID NO: 2 der Position 831 entspricht.

Das besagte Nukleinsäuremolekül kann dadurch gekennzeichnet sein, dass das Nukleinsäuremolekül an denjenigen Stellen, die den Stellen in der Referenz-Nukleotidsequenz SEQ ID NO: 1 entsprechen, eine oder mehrere der folgenden Nukleotidsubstitutionen aufweist:
(a) C statt A an Position 919;
(b) G statt A an Position 1310;
(c) A statt G an Position 1697;
(d) A statt C an Position 2191; und/oder
(e) T statt C an Position 2491.

Das besagte Nukleinsäuremolekül kann dadurch gekennzeichnet sein, dass das Nukleinsäuremolekül ein Polypeptid mit einer Aminosäuresequenz gemäß SEQ ID NO: 4 kodiert und/oder die kodierende DNA-Sequenz gemäß SEQ ID NO: 3 umfasst.

Gemäß einem zweiten Aspekt betrifft die vorliegende Erfindung einen Vektor, der das oben genannte Nukleinsäuremolekül umfasst.

Gemäß einem dritten Aspekt betrifft die vorliegende Erfindung eine transgene Pflanzenzelle, vorzugsweise von einer Pflanze der Gattung Beta, welche das oben genannte Nukleinsäuremolekül als Transgen, optional unter der Kontrolle eines heterologen Promoters, oder den oben genannten Vektor umfasst.

Gemäß einem vierten Aspekt betrifft die vorliegende Erfindung eine transgene Pflanze, vorzugsweise der Gattung Beta, oder ein Teil davon, die eine Pflanzenzelle umfasst.

Gemäß einem fünften Aspekt betrifft die vorliegende Erfindung eine BNYVV-resistente Pflanze der Species *Beta vulgaris* oder ein Teil davon, in welcher das besagte Nukleinsäuremolekül endogen vorhanden ist, oder in welcher die eine oder mehrere Mutationen, wie oben definiert, in ein endogenes Nukleinsäuremolekül mit einer Nukleotidsequenz, die die Sequenz gemäß SEQ ID NO: 1 umfasst oder die mit der komplementären Sequenz zu SEQ ID NO: 1 unter stringenten Bedingungen hybridisiert, eingebracht worden sind, wobei die Pflanze oder ein Teil davon nicht zur Subspezies *Beta vulgaris subsp. maritima* gehört.

Die oben genannte Pflanze ist eine Hybridpflanze oder doppelhaploide Pflanze, und/oder das Nukleinsäuremolekül oder die eine oder mehrere Mutationen liegen heterozygot oder homozygot vor.

Gemäß einem sechsten Aspekt betrifft die vorliegende Erfindung einen Samen von der oben genannten Pflanze, wobei der Samen ein oder mehrere der vorgenannten Nukleinsäuremoleküle oder Vektoren transgen oder endogen umfasst.

Gemäß einem siebten Aspekt betrifft die vorliegende Erfindung ein Verfahren zur Herstellung einer BNYVV-resistenten Pflanze, umfassend die folgenden Schritte:
(a) Mutagenisieren von Pflanzenzellen und anschließendem Regenerieren von Pflanzen aus den mutagenisierten Pflanzenzellen oder Mutagenisieren von Pflanzen, und
(b) Identifizieren einer Pflanze aus (a), welche in einem endogenen Nukleinsäuremolekül die eine oder mehrere Mutationen aufweist.

Gemäß einem achten Aspekt betrifft die vorliegende Erfindung ein Verfahren zur Identifikation einer Pflanze der Gattung Beta, die resistent gegenüber dem Pathogen BNYVV ist, dadurch gekennzeichnet, dass das Verfahren den nachfolgenden Schritt umfasst:
(i) Nachweisen des Vorhandenseins eines besagten Nukleinsäuremoleküls, wobei das davon kodierte Polypeptid (i) ein Glutamin (Q) an derjenigen Position aufweist, welche in der Referenz-Aminosäuresequenz SEQ ID NO: 2 der Position 307 entspricht, oder (ii) ein Arginin (R) an derjenigen Position aufweist, welche in der Referenz-Aminosäuresequenz SEQ ID NO: 2 der Position 437 entspricht, und/oder
   der Expression des Nukleinsäuremoleküls, wobei das Nukleinsäuremolekül an denjenigen Stellen, die den Stellen in der Referenz-Nukleotidsequenz SEQ ID NO: 1 entsprechen, die folgenden Nukleotidsubstitutionen aufweist: (a) C statt A an Position 919, oder (b) G statt A an Position 1310; in der Pflanze bzw. einer Probe davon; und/oder
(ii) Nachweisen des Vorhandenseins eines besagten Nukleinsäuremoleküls, wobei das davon kodierte Polypeptid (i) ein Glutamin (Q) an derjenigen Position aufweist, welche in der Referenz-Aminosäuresequenz SEQ ID NO: 2 der Position 307 entspricht, und (ii) ein Arginin (R) an derjenigen Position aufweist, welche in der Referenz-Aminosäuresequenz SEQ ID NO: 2 der Position 437 entspricht, und/oder
   der Expression des besagten Nukleinsäuremoleküls, wobei das Nukleinsäuremolekül an denjenigen Stellen, die den Stellen in der Referenz-Nukleotidsequenz SEQ ID NO: 1 entsprechen, die folgenden Nukleotidsubstitutionen aufweist: (a) C statt A an Position 919, und (b) G statt A an Position 1310; in der Pflanze bzw. einer Probe davon, und gegebenenfalls
(iii) Selektieren der BNYVV resistenten Pflanze.

Gemäß einem neunten Aspekt betrifft die vorliegende Erfindung eine Population von Pflanzen umfassend die oben genannten Pflanzen oder Pflanzen, welche durch das oben genannte Verfahren identifiziert und gegebenenfalls selektiert wurden.

Gemäß einem zehnten Aspekt betrifft die vorliegende Erfindung ein Verfahren zur Kontrolle des Befalls mit dem Pathogen Beet Necrotic Yellow Vein Virus (BNYVV) im agrarkulturellen oder hortikulturellen Anbau von Pflanzen der Gattung Beta, umfassend
I) das Identifizieren und Selektieren von Pflanzen der Gattung Beta mit Hilfe des oben genannten Verfahrens und
II) das Anbauen der Pflanzen aus I) oder Nachkommen davon.

Zunächst werden einige der in dieser Anmeldung verwendeten Begriffe nachfolgend näher erläutert:
Der Begriff "etwa" im Zusammenhang mit der Angabe einer Länge einer Nukleotidsequenz bedeutet eine Abweichung um +/- 10.000 Basenpaaren, +/- 5000 Basenpaaren oder +/- 1000 Basenpaaren, bevorzugt um +/- 200 Basenpaaren oder +/- 100 Basenpaaren und besonders bevorzugt um +/- 50 Basenpaaren.

Eine "Pflanze der Gattung Beta" gehört zu der Familie der Fuchsschwanzgewächse (Amaranthaceae). Zu diesen Pflanzen zählen Pflanzen der Spezies *Beta macrocarpa*, *Beta vulgaris, Beta lomatogona, Beta macrorhiza*, *Beta corolliflora*, *Beta trigyna* und *Beta nana.* Eine Pflanze der Spezies *Beta vulgaris* ist insbesondere eine Pflanze der Subspezies *Beta vulgaris* subsp. *vulgaris.* Hierzu zählen beispielsweise *Beta vulgaris* subsp. *vulgaris* var. *altissima* (Zuckerrübe i.e.S.), *Beta vulgaris* ssp. *vulgaris* var. *vulgaris* (Mangold), *Beta vulgaris* ssp. *vulgaris* var. *conditiva* (Rote Rübe/Bete), *Beta vulgaris* ssp. *vulgaris* var. *crassa*/*alba* (Futterrübe).

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. Basenpaarungen mit diesem eingeht. Standardverfahren zur Hybridisierung sind beispielsweise in Sambrook et al., Molecular Cloning: A Laboratory Manual 3rd Ed. Cold Spring Harbor Laboratory Press, Cold Spring Harbor, NY, 2001 beschrieben. Vorzugsweise wird darunter verstanden, dass mindestens 60 %, weiter bevorzugt mindestens 65 %, 70 %, 75 %, 80 % oder 85 %, besonders bevorzugt 90 %, 91 %, 92 %, 93 %, 94 %, 95 %, 96 %, 97 %, 98 % oder 99 % der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70 %, vorzugsweise mindestens 75 %, mindestens 80 %, mindestens 85 %, mindestens 90 % oder mindestens 95 % Sequenzidentität aufweisen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

"Komplementäre" Nukleotidsequenz bedeutet bezogen auf eine Nukleinsäure in Form einer doppelsträngigen DNA, dass der zum ersten DNA Strang komplementäre zweite DNA Strang entsprechend den Basenpaarungsregeln die Nukleotide aufweist, die zu den Basen des ersten Stranges korrespondieren.

Unter einem "isolierten Nukleinsäuremolekül" wird ein aus seiner natürlichen bzw. ursprünglichen Umgebung herausgelöstes Nukleinsäuremolekül verstanden. Der Begriff umfasst auch ein synthetisch hergestelltes Nukleinsäuremolekül. Unter einem "isolierten Polypeptid" wird ein aus seiner natürlichen bzw. ursprünglichen Umgebung herausgelöstes Polypeptid verstanden. Der Begriff umfasst auch ein synthetisch hergestelltes Polypeptid.

Ein "molekularer Marker" ist eine Nukleinsäure, die polymorph in einer pflanzlichen Population ist und als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Somit ist ein solcher Marker in der Lage verschiedene allelische Zustände (Allele) zu detektieren und zu unterscheiden. Der Begriff "molekularer Marker" bezieht sich auch auf Nukleotidsequenzen, welche komplementär oder zumindest weitestgehend komplementär oder homolog sind zu genomischen Sequenzen, zum Beispiel Nukleinsäuren welche als Sonden oder Primer eingesetzt werden. Für Marker finden sich diese Unterschiede auf DNA-Ebene und sind beispielsweise Polynukleotidsequenzunterschiede wie zum Beispiel SSRs (*simple sequence repeats*), RFLPs (*restriction fragment length polymorphisms*), FLPs (*fragment length polymorphisms*) oder SNPs (*single nucleotide polymorphisms*)*.* Die Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, die genetische Polymorphismen (zwischen Teilen einer Population) beschreiben, können unter Verwendung gut etablierter Verfahren aus dem Stand der Technik nachgewiesen werden (An Introduction to Genetic Analysis. 7th Edition, Griffiths, Miller, Suzuki et al., 2000). Dazu gehören z.B. DNA-Sequenzierung, PCR-basierte sequenzspezifische Amplifikation, Nachweis von RFLPs, Nachweis von Polynukleotid-Polymorphismen mittels Allel-spezifischer Hybridisierung (ASH), Detektion von amplifizierten variablen Sequenzen des Pflanzengenoms, Detektion einer 3SR (*self-sustained sequence replication*), Detektion von SSRs, SNPs, RFLPs oder AFLPs (*amplified fragment length polymorphisms*)*.* Ferner sind auch die Methoden zur Detektion von ESTs (*expressed sequence tags*) und SSR-Marker abgeleitet von EST-Sequenzen und RAPD (*randomly amplified polymorphie DNA*) bekannt. Je nach Kontext kann der Begriff Marker in der Beschreibung auch eine spezifische Chromosom-Position in dem Genom einer Spezies, wo ein spezifischer Marker (z.B. SNP) gefunden werden kann, meinen.

Ein "Promotor" ist eine nicht-translatierte regulatorische DNA-Sequenz, typischerweise stromaufwärts einer kodierenden Region, welche die Bindestelle für die RNA-Polymerase beinhaltet und die Transkription der DNA initiiert. Ein Promotor enthält zudem andere Elemente, die als Regulatorgen der Genexpression fungieren (z.B. cis-regulatorischen Elemente). Ein "Kern-oder Minimalpromotor" ist ein Promoter, der zumindest die Grundelemente, welche für die Transkriptionsinitiation gebraucht werden, aufweist (z.B. TATA-Box und/oder Initiator).

Ein "Pathogen" meint einen Organismus, der in Interaktionen mit einer Pflanze zu Krankheitssymptomen an einem oder mehreren Organen bei der Pflanze führt. Zu diesen Pathogenen zählen beispielsweise tierische, pilzliche, bakterielle oder virale Organismen oder Oomyceten.

Unter einer "Pathogeninfektion" ist der früheste Zeitpunkt zu verstehen, zu dem ein Pathogen mit einem pflanzlichen Wirtsgewebe interagiert. Beispielsweise im Fall des viralen Pathogens BNYVV wird dieser durch den Protozoen *Polymyxa betae* übertragen. *Polymyxa* bildet Sporen, die in der Erde über viele Jahrzehnte überdauern können. In diesen Sporen überdauert auch der Virus. Wenn diese Dauersporen zu mobilen Zoosporen auskeimen, kann der Virus über diese in Zellen des pflanzlichen Wirtsgewebes gelangen und dort mit dem Wirt interagieren (Esser (2000) Kryptogamen 1: Cyanobakterien Algen Pilze Flechten Praktikum und Lehrbuch. Springer-Verlag, Berlin, Heidelberg, 3. Auflage).

Pflanzliche "Organe" meinen beispielsweise Blätter, Sprossachse, Stamm, Wurzeln, Hypocotyl, vegetative Knospen, Meristeme, Embryos, Antheren, Ovula, Samenkörner oder Früchte. "Pflanzliche Teile" bzw. "Pflanzenteil(e)" beinhalten, sind jedoch nicht beschränkt auf die Sprossachse bzw. den Halm, Blätter, Blüten, Blütenstände, Wurzeln, Früchte und Samen sowie die Pollen. Der Begriff "Pflanzliche Teile" bzw. "Pflanzenteil(e)" meint ferner einen Zusammenschluss mehrerer Organe z.B. eine Blüte oder ein Samen, oder einen Teil eines Organs, z.B. eine Querschnitt durch die Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe sowie das Bildungsgewebe, Grundgewebe (das sogenannte Parenchym), Leitgewebe, Festigungsgewebe und das Deckgewebe (die sogenannte Epidermis). Das Gewebe wird durch diese Auflistung jedoch nicht beschränkt. Unter pflanzlichen "Zellen" sind beispielsweise isolierte Zellen mit einer Zellwand oder Aggregate davon oder Protoplasten zu verstehen.

Im Zusammenhang mit der vorliegenden Erfindung betrifft der Begriff "regulatorische Sequenz" eine Nukleotidsequenz, welche die Spezifität und/oder die Expressionsstärke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

Der Begriff "Resistenz" ist breit zu verstehen und deckt den Bereich des Schutzes von einer Verzögerung bis zur kompletten Hemmung der Entwicklung der Krankheit ab. Ein Beispiel für einen Pathogen von Bedeutung ist der Beet Necrotic Yellow Vein Virus (BNYVV). Vorzugsweise erreicht eine resistente Pflanzenzelle der Erfindung oder resistente Pflanze der Erfindung eine Resistenz gegen BNYVV. Eine Resistenz gegenüber einem Pathogen ist gleichzusetzen mit einer Resistenz gegenüber der Krankheit, welche dieser Pathogen verursacht, beispielsweise ist eine Resistenz gegenüber BNYVV auch eine Resistenz gegenüber Wurzelbärtigkeit (Rizomania).

"Transgene Pflanze" bezieht sich auf eine Pflanze, in deren Genom mindestens ein Polynukleotid integriert ist. Dabei kann es sich um ein heterologes Polynukleotid handeln. Bevorzugt ist das Polynukleotid stabil integriert, was bedeutet, dass das integrierte Polynukleotid in der Pflanze stabil erhalten bleibt, exprimiert wird und auch stabil an die Nachkommen vererbt werden kann. Das stabile Einbringen eines Polynukleotids in das Genom einer Pflanze schließt auch die Integration in das Genom einer Pflanze der vorhergehenden Parentalgeneration mit ein, wobei das Polynukleotid stabil weitervererbt werden kann. Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Ausgestaltungen und Ausführungsformen der vorliegenden Erfindung werden in exemplarischer Weise mit Bezug auf die anhängigen Sequenzen und Abbildungen beschrieben.

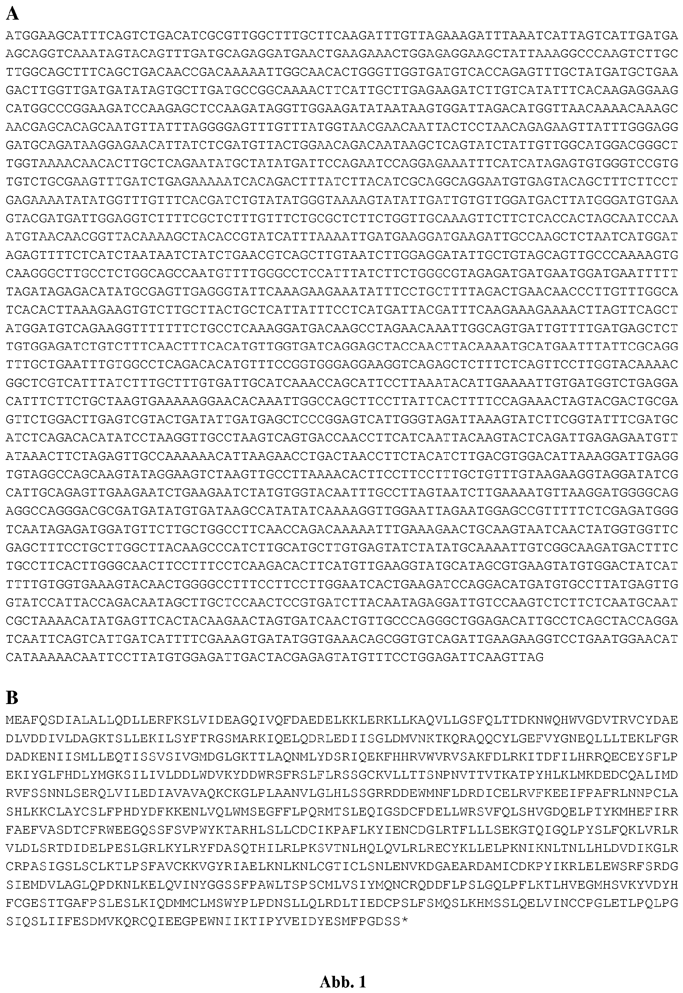

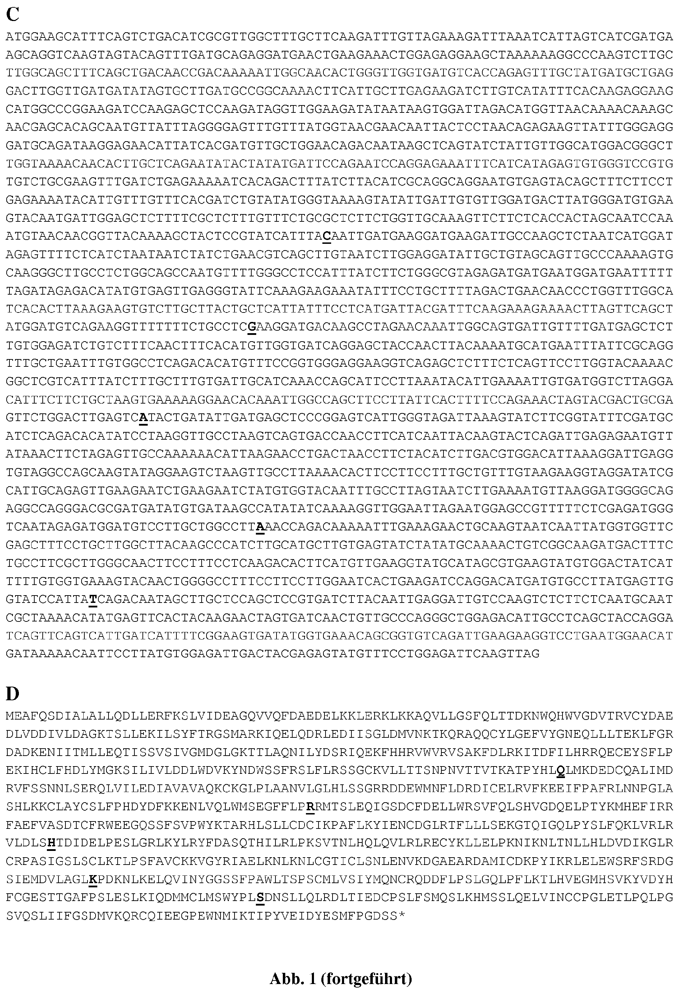

- **Abb. 1:**: **(A)** Genomische DNA-Sequenz des Wurzelbärtigkeit (WB) sensitiven Allels *wb*-s des Resistenzgens *wb* (SEQ ID NO: 1) aus einem ss-Genotyp, das als NBS-LRR (NB-ARC domain Gen) mittels Feinkartierung identifiziert wurde. Die Analyse der Nachkommenschaft von den 4 engsten rekombinanten Pflanzen (2 direkte Rekombinanten links und zwei direkte Rekombinanten rechts um das Gen herum) haben das NBS-LRR Gen punktgenau bis auf ein Gen eingegrenzt. Die gezeigte Sequenz umfasst die kodierende Region der vorhergesagten Proteinsequenz (**B,** SEQ ID NO: 2) des Resistenzproteins WB-s. **(C)** Genomische DNA-Sequenz eines WB resistenten Allels *wb*-R des Resistenzgens *wb* (SEQ ID NO: 3) umfassend die kodierende Region der vorhergesagten Proteinsequenz. Diagnostische Polymorphismen, die zu Aminosäureaustausche führen sind unterstrichen und fett gedruckt hervorgehoben. **(D)** Aminosäuresequenz eines von der genomischen DNA-Sequenz eines WB resistenten Allels *wb-*R des Resistenzgens *wb* (SEQ ID NO: 3) kodierten Proteins. Diagnostische Polymorphismen sind unterstrichen und fett gedruckt hervorgehoben. Experimente im Rahmen der vorliegenden Erfindung, u.a. Sequenzanalysen der WB resistenten Genotypen *versus* des vorherrschenden sensitiven Genotypen und in der Genomdatenbank enthaltenen Aminosäuresequenz ergaben, dass in der genomischen DNA-Sequenz der WB resistenten *wb*-R Allele das korrelierende NBS-LRR Gen mit dem in der SEQ ID NO: 3 gezeigten Leseraster ein oder mehrere Aminosäureaustausche aufweist, so die beobachtete Resistenz auf Mutationen in diesem Gen zurückzuführen sind. Aufgrund der bisherigen Analyse der Rekombinanten kann insbesondere einer der beiden Aminosäureaustausche K307Q und/oder Q437R als kausal für die Resistenz betrachtet werden.

### AUSFÜHRLICHE BESCHREIBUNG DER ERFINDUNG

Die vorliegende Erfindung betrifft ein Nukleinsäuremolekül, das in der Lage ist, eine Resistenz gegenüber einem Pathogen, insbesondere gegenüber dem "Beet Necrotic Yellow Vein Virus" (BNYVV) in einer Pflanze, insbesondere der Gattung Beta zu vermitteln, in welcher das Polypeptid, welches durch das Nukleinsäuremolekül kodiert wird, exprimiert wird. Die vorliegende Erfindung basiert auf der genetischen Feinkartierung, Identifizierung, Isolierung und Charakterisierung eines Gens, das ursprünglich aus dem Donor *Beta vulgaris* subsp. *maritima* stammt, dessen Vorhandensein in einer Pflanze, insbesondere in der Zuckerrübe mit der Resistenz der betreffenden Pflanze gegen Wurzelbärtigkeit (WB) korreliert bzw. ursächlich ist, und dessen Nukleotid- und kodierte Aminosäuresequenz sich durch mindestens einen Nukleotid- bzw. Aminosäureaustausch gegenüber der in Figur 1A und B gezeigten Nukleotid- und Aminosäuresequenz des identifizierten NBS-LRR Gens auszeichnet.

Gemäß dem ersten Aspekt betrifft die vorliegende Erfindung ein Nukleinsäuremolekül, welches ein Polypeptid kodiert, das in der Lage ist, eine Resistenz gegenüber einem Pathogen in einer Pflanze, in welcher das Polypeptid exprimiert wird, zu vermitteln, dadurch gekennzeichnet, dass das Nukleinsäuremolekül eine Nukleotidsequenz umfasst, welche ausgewählt ist aus
(a) einer Nukleotidsequenz, die ein Polypeptid mit einer Aminosäuresequenz, die zu mindestens 80 % identisch mit der SEQ ID NO: 2 ist, kodiert, wobei das Nukleinsäuremolekül eine oder mehrere Mutationen aufweist, welche dazu führen, dass das Polypeptid, das durch das Nukleinsäuremolekül kodiert wird, an derjenigen Position, welche in der Referenz-Aminosäuresequenz SEQ ID NO: 2 der Position 307 entspricht, eine Aminosäure aufweist, welche sich von Lysin (K) unterscheidet, und/oder an derjenigen Position, welche in der Referenz-Aminosäuresequenz SEQ ID NO: 2 der Position 437 entspricht, eine Aminosäure aufweist, welche sich von Glutamin (Q) unterscheidet; und
(b) einer Nukleotidsequenz, die mit der komplementären Sequenz zu SEQ ID NO: 1 unter stringenten Bedingungen hybridisiert, wobei die Nukleotidsequenz an denjenigen Stellen, die den Stellen in der Referenz-Nukleotidsequenz SEQ ID NO: 1 entsprechen, eine oder mehrere der folgenden Nukleotidsubstitutionen aufweist: C statt A an Position 919; G statt A an Position 1310; A statt G an Position 1697; A statt C an Position 2191; und/oder T statt C an Position 2491;
vorzugsweise wobei das Pathogen das Beet necrotic yellow vein virus (BNYVV) und die Pflanze eine Pflanze der Gattung Beta, vorzugsweise Zuckerrübe (Beta vulgaris L.), ist.

Bei dem Nukleinsäuremolekül kann es sich um ein isoliertes Nukleinsäuremolekül handeln. Bevorzugt handelt es sich um DNA, und besonders bevorzugt um cDNA bzw kodierende DNA. Vorzugsweise vermittelt das Polypeptid, welches durch das erfindungsgemäße Nukleinsäuremolekül kodiert wird, eine Resistenz gegenüber dem viralen Pathogen *"Beet Necrotic Yellow Vein Virus"* (BNYVV), welcher die Pflanzenkrankheit Wurzelbärtigkeit (Rizomania) verursacht und durch den bodenbürtigen Protozoen *Polymyxa betae* übertragen wird. Weiterhin vermittelt das Polypeptid, welches durch das erfindungsgemäße Nukleinsäuremolekül kodiert wird, insbesondere in einer Pflanze der Gattung Beta eine Resistenz gegenüber diesem Pathogen. Bevorzugt handelt es sich bei der Pflanze um eine Pflanze der Spezies *Beta vulgaris,* besonders bevorzugt um eine Pflanze der Subspezies *Beta vulgaris* subsp. *vulgaris*; hierzu zählen beispielsweise die Kulturarten Zuckerrübe, Rote Bete, Futterrübe, Blattmangold und Stielmangold.

In einer Ausführungsform der vorliegenden Erfindung umfasst das erfindungsgemäße Nukleinsäuremolekül eine Nukleotidsequenz, die ein Polypeptid mit einer Aminosäuresequenz, die zu mindestens 80 % identisch mit der SEQ ID NO: 2 ist, kodiert,, wobei das Nukleinsäuremolekül eine oder mehrere Mutationen aufweist, welche dazu führen, dass das Polypeptid, das durch das Nukleinsäuremolekül kodiert wird, an derjenigen Position, welche in der Referenz-Aminosäuresequenz SEQ ID NO: 2 der Position 307 entspricht, eine Aminosäure aufweist, welche sich von Lysin (K) unterscheidet, und/oder an derjenigen Position, welche in der Referenz-Aminosäuresequenz SEQ ID NO: 2 der Position 437 entspricht, eine Aminosäure aufweist, welche sich von Glutamin (Q) unterscheidet. Eine der beiden oder beide erwähnten Mutationen in der Aminosäuresequenz sind mutmaßlich als kausal für die Resistenz gegen Rizomania identifiziert worden.

Wie in den Beispielen und in der Abbildungslegende erläutert, handelt es sich bei dem identifizierten Gen um ein Resistenzgen/-protein des Typs NBS-LRR, das durch bestimmte Strukturmotive charakterisiert ist. Die allgemeine Struktur von solchen Resistenzproteinen in Pflanzen ist bereits gut untersucht (Martin et al., Annual Review Plant Biology 54 (2003), 23-61). Jedoch ist das Prinzip der strukturellen Ausgestaltung insbesondere der sogenannten LRR-Domäne, welche als potentielle Erkennungsdomäne für zumeist unbekannte pathogene Effektoren gilt, nicht vorhersagbar und generell ist der funktionelle Hintergrund der Resistenzgene, d.h. die genetische Struktur, weitgehend unbekannt. Demzufolge ist die Identifizierung eines BNYVV-Resistenz-vermittelnden Gens bzw. Proteins allein auf Grundlage der bekannten Strukturmotive unmöglich. Weiterhin sind oftmals die Sequenzregionen um diese Resistenzgene herum repetitiv, was die Entwicklung von diagnostischen Markern sowie die Assemblierung von Sequenzen besonders schwierig macht.

Das identifizierte Resistenzprotein gehört zum Typ NBS-LRR und weist mindestens eine Leuzin-reiche Domäne (LRR) entsprechend den Aminosäurepositionen 558 bis 594 der SEQ ID NO: 4, bevorzugt den Aminosäurepositionen 542 bis 594 der SEQ ID NO: 4, den Aminosäurepositionen 604 bis 634 der SEQ ID NO: 4, bevorzugt den Aminosäurepositionen 582 bis 634 der SEQ ID NO: 4, den Aminosäurepositionen 760 bis 790 der SEQ ID NO: 4 oder den Aminosäurepositionen 838 bis 869 der SEQ ID NO: 4, und/oder mindestens eine AAA ATPase Domäne entsprechend den Aminosäurepositionen 177 bis 289 der SEQ ID NO: 4 oder den Aminosäurepositionen 156 bis 263 der SEQ ID NO: 4 kodiert, auf. Bei der identifizierten ATPase-Domäne handelt es sich höchstwahrscheinlich um die NB-ARC-Domäne, welcher eine zentrale Nukleotid-bindende Funktion zukommt. Ohne an eine bestimmt Theorie gebunden zu sein, reguliert diese funktionelle ATPase-Domäne voraussichtlich die Aktivität des Resistenzproteins.

In einer weiteren Ausführungsform umfasst das erfindungsgemäße Nukleinsäuremolekül eineNukleotidsequenz, die mit der komplementären Sequenz zu SEQ ID NO: 1 unter stringenten Bedingungen hybridisiert, wobei an den Positionen 919-921 und/oder 1309-1311 ein oder mehrere Nukleotide ausgetauscht sind, was zu einem Aminosäureaustausch führt.

Diese Aminosäureaustausche bzw. Nukleotidaustausche können unter Verwendung herkömmlicher Verfahren, die im Stand der Technik bekannt sind, beispielsweise durch ortsgerichtete Mutagenese, PCRvermittelte Mutagenese, Transposon-Mutagenese, TILLING, Genome Engineering beispielsweise mittels Meganukleasen, Zinkfingernukleasen, TALENS und CRISPR/Cas., etc durchgeführt werden. Des Weiteren können in die Nukleotidsequenz weitere Substitutionen, Deletionen, Insertionen, Additionen und/oder jede andere Änderung entweder allein oder in Kombinationen zusätzlich eingeführt werden, die die Nukleotidsequenz zwar ändern, jedoch die gleiche Funktion erfüllen, wie die Ausgangssequenz, hier die erfindungsgemäße Nukleotidsequenz, die ein Polypeptid mit der erfindungsgemäßen Aminosäuresequenz kodiert, welches Resistenz gegenüber Wurzelbärtigkeit (Rizomania) vermittelt. Daher umfasst die Erfindung in einer weiteren Ausführungsform eine Nukleotidsequenz, die ein Polypeptid kodiert, welches ein Derivat des Polypeptids darstellt, welches von der erfindungsgemäßen Nukleotidsequenz kodiert wird oder welches die erfindungsgemäße Aminosäuresequenz umfasst. Ein Derivat des Polypeptids stellt eine abgeleitete Aminosäuresequenz dar, welche mindestens eine Substitution, Deletion, Insertion oder Addition von einer oder mehrerer Aminosäuren aufweist, wobei die Funktionalität des kodierten Polypeptids/Proteins erhalten bleibt.

Bei der Substitution einer Aminosäure durch eine andere Aminosäure mit gleichen oder äquivalenten oder ähnlichen chemisch-physikalischen Eigenschaften spricht man von einem "konservativen Austausch" oder "semikonservativen Austausch". Beispiele für physikalisch-chemische Eigenschaften einer Aminosäure sind beispielsweise die Hydrophobie oder die Ladung. Dem Fachmann ist bekannt, welche Aminosäuresubstitution einen konservativen oder semikonservativen Austausch darstellt. Das allgemeine Fachwissen erlaubt es darüber hinaus, dass der Fachmann erkennen, identifizieren und nachweisen kann, welche Aminosäuredeletionen und -additionen für die Funktionalität des Resistenzproteins unschädlich sind und an welchen Positionen diese möglich sind. Dem Fachmann ist bewusst, dass im Falle des vorliegenden NBS-LRR-Proteins für Modifikationen der Aminosäuresequenz (Substitutionen, Deletionen, Insertion oder Additionen von einer oder mehrerer Aminosäuren) insbesondere die Funktionalität der oben definierten konservierten Domänen erhalten bleiben muss und dass daher in diesen Domänen nur begrenzt vorstehende Modifikationen möglich sind.

Somit umfasst die Erfindung ein funktionelles Fragment der erfindungsgemäßen Nukleotidsequenz. Der Begriff "Fragment" umfasst dabei Gene mit einer Nukleotidsequenz ausreichend ähnlich zu der oben genannten Nukleotidsequenz. Der Begriff "ausreichend ähnlich" bedeutet eine erste Nukleotidsequenz oder Aminosäuresequenz, die eine ausreichende oder minimale Anzahl an identischen oder äquivalenten Nukleotiden bzw. Aminosäureresten relativ zu einem zweiten Nukleotid bzw. einer zweiten Aminosäuresequenz hat.

Im Hinblick auf die Aminosäuresequenz weist diese auch nach Änderung durch ein oben genanntes Verfahren eine gemeinsame Strukturdomäne auf und/oder besitzt gemeinsame funktionelle Aktivität. Aminosäuresequenzen, die eine Identität von mindestens 80 %, mindestens 85 %, mindestens 90 %, mindestens 91 %, mindestens 92 %, mindestens 93 %, mindestens 94 %, mindestens 95 %, mindestens 96 %, mindestens 97 %, mindestens 98 %, mindestens 99 %, oder 100 % zu der erfindungsgemäßen Nukleotidsequenz bzw. Aminosäuresequenz aufweisen, werden hier als ausreichend ähnlich definiert. Vorzugsweise können solche Nukleotidsequenzen, welche für ein Derivat bzw. für eine abgeleitete Aminosäuresequenz kodieren, entweder direkt oder indirekt (beispielsweise via Amplifikations- oder Replikationsschritte) aus einer Ausgangsnukleotidsequenz, welche über die Gesamtlänge oder zumindest teilweise der erfindungsgemäßen Nukleotidsequenz, erzeugt werden.

Entsprechend umfasst die vorliegende Erfindung eine Nukleotidsequenz, die in der Lage ist, an eine Nukleotidsequenz, die komplementär zu einer erfindungsgemäßen Nukleotidsequenz bzw. zu der Nukleotidsequenz, die die erfindungsgemäße Aminosäuresequenz kodiert, unter stringenten Bedingungen zu hybridisieren.

In einer weiteren Ausführungsform umfasst das erfindungsgemäße Nukleinsäuremolekül eine Nukleotidsequenz, wobei das Nukleinsäuremolekül eine oder mehrere Mutationen aufweist, welche dazu führen, dass das Polypeptid, das durch das Nukleinsäuremolekül kodiert wird, an derjenigen Position, welche in der Referenz-Aminosäuresequenz SEQ ID NO: 2 der Position 307 entspricht, eine Aminosäure aufweist, welche sich von Lysin (K) unterscheidet, und/oder an derjenigen Position, welche in der Referenz-Aminosäuresequenz SEQ ID NO: 2 der Position 437 entspricht, eine Aminosäure aufweist, welche sich von Glutamin (Q) unterscheidet und an derjenigen Position, welche in der Referenz-Aminosäuresequenz SEQ ID NO: 2 der Position 566 entspricht, eine Aminosäure aufweist, welche sich von Arginin (R) unterscheidet, und/oder (ii) an derjenigen Position, welche in der Referenz-Aminosäuresequenz SEQ ID NO: 2 der Position 731 entspricht, eine Aminosäure aufweist, welche sich von Glutamin (Q) unterscheidet, und/oder (iii) an derjenigen Position, welche in der Referenz-Aminosäuresequenz SEQ ID NO: 2 der Position 831 entspricht, eine Aminosäure aufweist, welche sich von Prolin (P) unterscheidet. Die ersten beiden Austausche wurden wie vorstehend erwähnt als verantwortlich für die Resistenz identifiziert. Obwohl die Rolle der letzten drei Austausche bei der Resistenzvermittlung noch unklar ist, sind sie diagnostisch und können somit vorteilhaft in Nachweis- und/oder Selektionsverfahren einsetzen werden..

In einer weiteren Ausführungsform umfasst das erfindungsgemäße Nukleinsäuremolekül eine Nukleotidsequenz, wobei das kodierte Polypeptid ein Glutamin (Q) an derjenigen Position aufweist, welche in der Referenz-Aminosäuresequenz SEQ ID NO: 2 der Position 307 entspricht, und/oder ein Arginin (R) an derjenigen Position aufweist, welche in der Referenz-Aminosäuresequenz SEQ ID NO: 2 der Position 437 entspricht, und/oder ein Histidin (H) an derjenigen Position aufweist, welche in der Referenz-Aminosäuresequenz SEQ ID NO: 2 der Position 566 entspricht, und/oder ein Lysin (K) an derjenigen Position aufweist, welche in der Referenz-Aminosäuresequenz SEQ ID NO: 2 der Position 731 entspricht, und/oder ein Serin (S) an derjenigen Position aufweist, welche in der Referenz-Aminosäuresequenz SEQ ID NO: 2 der Position 831 entspricht. Diese Aminosäureaustasche kommen bevorzugt durch Substitution einer oder mehrerer Nukleotide in dem Nukleinsäuremolekül der SEQ ID NO: 1 zustande, wobei bevorzugt ein Nukleotid durch ein anderes substituiert wird. Dementsprechend ist das erfindungsgemäße Nukleinsäuremolekül dadurch gekennzeichnet, dass das Nukleinsäuremolekül an denjenigen Stellen, die den Stellen in der Referenz-Nukleotidsequenz SEQ ID NO: 1 entsprechen, eine oder mehrere der folgenden Nukleotidsubstitutionen aufweist: C statt A an Position 919; G statt A an Position 1310; A statt G an Position 1697; A statt C an Position 2191; und/oder T statt C an Position 2491. In einer bevorzugten Ausführungsform ist das erfindungsgemäße Nukleinsäuremolekül dadurch gekennzeichnet ist, dass es ein Polypeptid mit einer Aminosäuresequenz gemäß SEQ ID NO: 4 kodiert und/oder die kodierende DNA-Sequenz gemäß SEQ ID NO: 3 umfasst; siehe auch Abbildung 1 und die Figurlegende dazu sowie die Beispiele.

Weiterhin offenbart die vorliegende Anmeldung ein rekombinantes DNA-Molekül, das die Sequenzen des besagten Nukleinsäuremoleküls umfasst. Vorzugsweise weist das rekombinante DNA-Molekül weiterhin eine regulatorischen Sequenz auf bzw. ist mit dieser assoziiert/operativ verknüpft, vorzugsweise mit einer Promotorsequenz und/oder anderen Sequenzen von Transkriptions- oder Translationskontrollelementen, wobei die regulatorische Sequenz, welche die Expression eines Gens, das das erfindungsgemäße Nukleinsäuremolekül umfasst, kontrolliert, dadurch gekennzeichnet ist, dass die regulatorische Sequenz in der Lage ist, die Expression einer heterologen DNA-Sequenz infolge einer Pathogeninfektion zu vermitteln oder zu modulieren. Vorzugsweise ist die heterologe DNA-Sequenz eine Nukleotidsequenz, welche für eine Komponente der pflanzlichen Pathogenabwehr kodiert (Bsp.: Resistenzgene (R-Gene) oder Gene, welche für am Signaltransfer beteiligte Enzyme wie Kinasen oder Phosphatasen sowie für G-Protein kodieren) oder welche für einen pathogenen Effektor kodiert (sog. Avirulenzgene (*avr*))*.*

Die vorliegende Erfindung offenbart weiterhin auch ein Polypeptid, welches durch das erfindungsgemäße Nukleinsäuremolekül kodiert ist und ein funktionelles und/oder immunologisch aktives Fragment davon sowie einen Antikörper, der spezifisch an das Polypeptid oder an dessen Fragment bindet. Besonders bevorzugt weist das Polypeptid eine Aminosäuresequenz gemäß SEQ ID NO: 4 auf. Die rekombinante Herstellung von Proteinen, Polypeptiden und Fragmenten ist dem Fachmann geläufig und beispielsweise in Sambrook et al., Molecular Cloning: A Laboratory Manual 3rd Ed. Cold Spring Harbor Laboratory Press, Cold Spring Harbor, NY, 2001 oder Wingfield, P. T. 2008. Production of Recombinant Proteins. Current Protocols in Protein Science. 52:5.0:5.0.1-5.0.4 beschrieben. Polyklonale oder monoklonaler Antikörper zu dem Protein der vorliegenden Erfindung können von dem Fachmann nach bekannten Verfahren hergestellt werden, wie sie beschrieben sind in E. Harlow et al., Herausgeber Antibodies: A Laboratory Manual (1988). Die Herstellung von monoklonalen Antikörpern sowie von Fab- und F(ab')₂-Fragmenten, die auch bei Proteindetektionsmethoden nützlich sind, kann durchgeführt werden durch verschiedene gebräuchliche Methoden wie sie beschrieben sind in Goding, Mononoclonal Antibodies: Principles and Practice, S. 98-118, New York: Academic Press (1983). Der Antikörper kann dann für das Screening von Expressions-cDNA-Bibliotheken genutzt werden um identischen, homologe oder heterologe Gene mittels immunologischen Screening zu identifizieren (Sambrook et al., Molecular Cloning: A Laboratory Manual, Cold Spring Harbor Laboratory Press, Cold Spring Harbor, NY, 1989 oder Ausubel et al., 1994, "Current Protocols in Molecular Biology", John Wiley & Sons). Insbesondere betrifft die vorliegende Erfindung Antiköper, die ein durch das erfindungsgemäße *wb*-R Allel kodiertes Polypeptid selektiv erkennen und im Wesentlichen nicht das vom sensitiven *wb*-s Allel kodierte Polypeptide, d.h. mindestens um einen Faktor 2, vorzugsweise Faktor 5, und mehr bevorzugt um einen Faktor 10 oder mehr geringer als das durch das erfindungsgemäße *wb*-R Allel kodierte Polypeptid.

Ein weiterer Gegenstand der Erfindung sind Vektoren, die das erfindungsgemäße Nukleinsäuremolekül bzw. das rekombinante DNA-Molekül umfassen. Bei dem Vektor kann es sich um ein Plasmid, ein Cosmid, eine Phage oder einen Expressionsvektor, einen Transformationsvektor, Shuttle-Vektor oder Klonierungsvektor handelt, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße Nukleinsäuremolekül oder DNA-Molekül in einem Expressionsvektor mit einer oder mehreren regulatorischen Sequenzen, welche die Transkription und optional die Expression in einer prokaryotischen oder eukaryotischen Wirtszelle erlauben, operativ verknüpft; siehe beispielsweise Sambrook et al., Molecular Cloning: A Laboratory Manual 3rd Ed. Cold Spring Harbor Laboratory Press, Cold Spring Harbor, NY, 2001. Vorzugsweise sind diese regulatorischen Sequenzen Promotoren oder Terminatoren insbesondere ein Transkriptions-Initiations-Startpunkt, eine Ribosomen-Bindestelle, ein RNA-prozessierendes Signal, eine Transkriptions-Terminations Stelle und/oder ein Polyadenylierungssignal. Beispielsweise steht hierbei das Nukleinsäuremolekül unter der Kontrolle eines geeigneten Promotors und/oder eines Terminators. Geeignete Promotoren können Promotoren sein, welche konstitutiv induziert werden (Bsp.: 35S-Promoter aus dem "Cauliflower mosaic virus" (Odell et al., Nature 313 (1985), 810 - 812), besonders geeignet sind solche Promotoren, welche pathogen-induzierbar sind (Bsp.: PR1-Promotor aus Petersilie (Rushton et al., EMBO J. 15 (1996), 5690-5700). Besonders geeignete Pathogen-induzierbare Promotoren sind synthetische bzw. chimäre Promotoren, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren werden den gewünschten Anforderungen nach konzipiert und werden durch unterschiedliche Faktoren induziert oder reprimiert. Beispiele für solche Promotoren finden sich in WO 00/29592, WO 2007/147395 und WO 2013/091612. Ein geeigneter Terminator ist beispielsweise der nos-Terminator (Depicker et al., J. Mol. Appl. Genet. 1 (1982), 561-573). Die Vektoren enthalten zusätzlich für gewöhnlich Indikator-/Reportergene oder Resistenzgene zum Nachweisen der Übertragung des gewünschten Vektors bzw. DNA-Moleküls/Nukleinsäuremoleküls und zum Selektieren der Individuen die diese enthalten, da ein direkter Nachweis über die Expression des Gens meinst eher schwierig ist. Da das erfindungsgemäße Nukleinsäuremolekül hier selber ein Polypeptid kodiert, welches mit den zuvor erwähnten Mutationen das Protein darstellt, welches Resistenz gegen Rizomania verleiht, ist es nicht essentiell für die Expression in Pflanzenzellen, ein weiteres Resistenzgen bereit zu stellen, wird aber bevorzugt bereitgestellt, um eine schnelle Selektion zu erlauben.

Beispiele für Indikator-/Reportergene sind beispielsweise das Luciferase-Gen und das Gen kodierend für das Grün-fluoreszierende-Protein (GFP). Diese erlauben ferner auch Untersuchungen zur Aktivität und/oder Regulation eines Promotors des Gens. Beispiele für Resistenzgene, im speziellen für Pflanzentransformationen sind das Neomycin-Phosphotransferase-Gen, das Hygromycin-Phosphotransferase Gen oder das Gen kodierend für die Phosphinothricin-Acetyltransferase. Dies schließt weitere dem Fachmann bekannte Indikator-/Reportergene oder Resistenzgene jedoch nicht aus. In einer bevorzugten Ausführungsform ist der Vektor ein Pflanzenvektor.

Im Weiteren offenbart die vorliegende AnmeldungWirtszellen, die die erfindungsgemäßen Vektoren, rekombinante DNA-Moleküle und/oder Nukleinsäuremoleküle umfassen. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle.

Dem Fachmann sind sowohl zahlreiche Methoden wie Konjugation oder Elektroporation bekannt, mit denen er das erfindungsgemäße Nukleinsäuremolekül, das rekombinante DNA-Molekül und/oder den Vektor der vorliegenden Erfindung in ein Agrobakterium einbringen kann, als auch Methoden wie diverse Transformationsverfahren (biolistische Transformation, Agrobakterium-vermittelte Transformation), mit denen er das erfindungsgemäße Nukleinsäuremolekül, das DNA-Molekül und/oder den Vektor der vorliegenden Erfindung in eine Pflanzenzelle einbringen kann (Sambrook et al., Molecular Cloning: A Laboratory Manual 3rd Ed. Cold Spring Harbor Laboratory Press, Cold Spring Harbor, NY, 2001).

Weiter bevorzugt betrifft die vorliegende Erfindung eine transgene Pflanzenzelle, die das erfindungsgemäße Nukleinsäuremolekül oder DNA-Molekül als Transgen oder den Vektor der vorliegenden Erfindung umfasst. Eine solche transgene Pflanzenzelle ist beispielsweise eine Pflanzenzelle, welche mit dem erfindungsgemäßen Nukleinsäuremolekül, DNA-Molekül oder mit dem Vektor der vorliegenden Erfindung, vorzugsweise stabil, transformiert ist. In einer bevorzugten Ausgestaltung der transgenen Pflanzenzelle ist das Nukleinsäuremolekül mit einer oder mehreren regulatorischen Sequenzen, welche die Transkription und optional die Expression in der Pflanzenzelle erlauben, operativ verknüpft. Das Gesamtkonstrukt aus dem erfindungsgemäßen Nukleinsäuremolekül und der/den regulatorischen Sequenzen stellt dann das Transgen dar. Solche regulatorische Sequenzen sind beispielsweise ein Promotor, ein Enhancer oder ein Terminator. Dem Fachmann sind zahlreiche in Pflanzen anwendbare, funktionelle Promotoren, Enhancer und Terminatoren bekannt.

Bevorzugt zeigt eine transgene Pflanzenzelle der vorliegenden Erfindung, insbesondere eine Zelle einer Pflanze der Gattung Beta, gegenüber einem Pathogen, insbesondere BNYVV, eine höhere Resistenz als eine korrespondierende nicht-transformierte Pflanzenzelle (die Pflanzenzelle ohne das Transgen). Das Level der Resistenz beispielsweise gegenüber BNYVV kann in Pflanzen der Gattung Beta qualitativ durch Bestimmung von Boniturnoten festgelegt werden (Boniturnotenschemata für Pflanze der Gattung Beta sind aus dem Stand der Technik bekannt, beispielsweise für Zuckerrüben; siehe Mechelke (1997) Probleme in der Rizomaniaresistenzzüchtung, Vorträge für Pflanzenzüchtung, Resistenzzüchtung bei Zuckerrüben, Gesellschaft für Pflanzenzüchtung e.V., 113-123). Eine höhere Resistenz zeigt sich in einer Verbesserung der Resistenz um mindestens eine Boniturnote, um mindestens zwei Boniturnoten, um mindestens drei oder mehr Boniturnoten. Weiterhin offenbart die vorliegende Anmeldung auch ein Verfahren zur Herstellung einer transgenen Pflanzenzelle der vorliegenden Erfindung, welches einen Schritt des Einbringens des erfindungsgemäßen Nukleinsäuremoleküls, DNA-Moleküls oder des Vektors der vorliegenden Erfindung in eine pflanzliche Zelle umfasst. Beispielsweise kann das Einbringen durch Transformieren stattfinden, vorzugsweise durch stabiles Transformieren. Die geeigneten Techniken zur Einbringung wie biolistische Transformation, Agrobakterium-vermittelte Transformation oder Elektroporation sind dem Fachmann bekannt (Sambrook et al., Molecular Cloning: A Laboratory Manual 3rd Ed. Cold Spring Harbor Laboratory Press, Cold Spring Harbor, NY, 2001).

In einem weiteren Aspekt betrifft die vorliegende Erfindung eine transgene Pflanze und Teile davon, umfassend eine vorstehend beschriebene transgene Pflanzenzelle. Ein Teil kann dabei eine Zelle, ein Gewebe, ein Organ oder ein Zusammenschluss von mehreren Zellen, Geweben, oder Organen sein. Ein Zusammenschluss von mehreren Organen ist z.B. eine Blüte oder ein Samen. In einer besonderen Ausgestaltung betrifft die Erfindung einen Samen von der transgenen Pflanze, wobei der Samen das erfindungsgemäße Nukleinsäuremolekül als Transgen umfasst. Bevorzugt zeigt eine transgene Pflanze der vorliegenden Erfindung, insbesondere eine Pflanze der Gattung Beta, gegenüber einem Pathogen, insbesondere BNYVV, eine höhere Resistenz als eine korrespondierende nicht-transformierte Pflanze (Pflanze ohne das Transgen). Das Level der Resistenz beispielsweise gegenüber BNYVV kann in Pflanzen der Gattung Beta qualitativ durch Bestimmung von Boniturnoten festgelegt werden (Boniturnotenschemata für Pflanze der Gattung Beta sind aus dem Stand der Technik bekannt, beispielsweise für Zuckerrüben Mechelke (1997) Probleme in der Rizomaniaresistenzzüchtung, Vorträge für Pflanzenzüchtung, Resistenzzüchtung bei Zuckerrüben, Gesellschaft für Pflanzenzüchtung e.V., 113-123). Eine höhere Resistenz zeigt sich in einer Verbesserung der Resistenz um mindestens eine Boniturnote, um mindestens zwei Boniturnoten, um mindestens drei oder mehr Boniturnoten. Ferner stellt die Erfindung ein Verfahren zur Herstellung einer transgenen Pflanze bereit, welches einen Schritt des Einbringens des erfindungsgemäßen Nukleinsäuremoleküls oder des Vektors der vorliegenden Erfindung in eine pflanzliche Zelle und optional einen Schritt der Selektion einer transgenen Pflanzenzelle umfasst. Weiterhin ist ein solches Verfahren zur Herstellung einer transgenen Pflanze durch einen anschließenden Schnitt gekennzeichnet, der die Regenerierung der transgenen Pflanze aus der im ersten Schritt erzeugten transgenen Pflanzenzelle einschließt. Methoden zur Regeneration sind dem Fachmann aus dem Stand der Technik bekannt.

In einem weiteren Aspekt betrifft die vorliegende Erfindung auch ein Verfahren zur Vermittlung oder Erhöhung einer Resistenz gegenüber einem Pathogen, insbesondere BNYVV, in einer Pflanze, bevorzugt einer Pflanze der Gattung Beta, welche einen Schritt des Transformierens einer Pflanzenzelle mit dem erfindungsgemäßen Nukleinsäuremolekül oder dem Vektor der vorliegenden Erfindung umfasst. Alternativ kann wie vorstehend beschrieben aus einem WB sensitiven Genotyp mittels zufälligem oder gezieltem Mutagenisieren des vorhandenen endogenen wb-s Gen ein WB resistenter Genotyp hergestellt werden.

Beispielweise kann das wb Gen durch Genmutation mittels TALE-Nukleasen (TALENs) oder ZinkfingerNukleasen (ZFNs) sowie CRISPR/Cas Systeme, die u.a. beispielhaft in WO 2014/144155 A1 (Engineering plant genomes using CRISPR/Cas systems) und in Osakabe & Osakabe, Plant Cell Physiol, 56 (2015), 389-400 beschrieben sind modifiziert werden. Dies kann auch durch Verwendung der als TILLING (*Targeted Induced Local Lesions in Genomes*) bezeichneten Methode erreicht werden, wobei, wie es beispielweise in der deutschen Patentanmeldung DE 10 2013 101 617 beschrieben ist, Punktmutationen in dem Wildtypgen hervorgerufen und anschließend Pflanzen selektiert werden, die eine geeignete, d.h. resistenzvermittelnde Mutation aufweisen, wie z.B. eine gegen Gelbmosaikvirose resistente Gerste; siehe die DE 10 2013 101617 auf Seiten 4, 8 und 12 in Paragraphen [0014], [0026] und [0038]. Die TILLING Methode ist auch ausführlich in der Publikation von Henikoff *et al.* beschrieben (Henikoff et al., Plant Physiol. 135, 2004, 630-636).

Bevorzugt führt dieses Verfahren zu einer Verbesserung der Resistenz um mindestens eine Boniturnote, besonders bevorzugt zu einer Verbesserung der Resistenz um mindestens zwei, drei oder mehr Boniturnoten. Boniturnotenschemata für Pflanze der Gattung Beta sind aus dem Stand der Technik bekannt, beispielsweise für Zuckerrüben Mechelke (1997). Nach dem Mutagenisieren der Pflanzenzellen und anschließendem Regenerieren von Pflanzen aus den mutagenisierten Pflanzenzellen oder Mutagenisieren von Pflanzen können dann die Pflanzen identifiziert werden, die in einem endogenen Nukleinsäuremolekül vorzugsweise mit der Nukleotidsequenz, die mit der komplementären Sequenz zu SEQ ID NO: 1 unter stringenten Bedingungen hybridisiert, die eine oder mehrere Mutationen wie vorstehend definiert aufweisen.

In einem weiteren Aspekt betrifft die vorliegende Erfindung eine BNYVV-resistente Pflanze der Gattung Beta oder ein Teil davon, in welcher das erfindungsgemäße Nukleinsäuremolekül endogen vorhanden ist, oder in welcher eine oder mehrere der vorstehend definierten Mutationen in ein endogenes, ansonsten mit dem WB sensitiven Genotyp korrelierenden Nukleinsäuremolekül, eingebracht worden sind, wobei die Pflanze oder ein Teil davon nicht zur Spezies *Beta vulgaris subsp. maritima* gehört. In einer Ausführungsform handelt es sich bei der erfindungsgemäßen Pflanze um eine Hybridpflanze oder eine doppelhaploide Pflanze. In einer weiteren Ausführungsform der erfindungsgemäßen Pflanze liegt das Nukleinsäuremolekül oder die eine oder mehrere Mutationen wie vorstehend definiert heterozygot oder homozygot vor. Im Falle beispielsweise einer Hybridpflanze kann das Nukleinsäuremolekül oder die eine oder mehrere Mutationen auch hemizygot vorliegen.

Im Weiteren offenbart die Anmeldung, dass die Pflanze zusätzlich transgen oder endogen ein zweites Nukleinsäuremolekül an einer anderen oder weiteren Position im Genom umfasst, welches ein Polypeptid kodiert, das in der Lage ist, eine Resistenz gegenüber BNYVV in der Pflanze, in welcher das Polypeptid infolge der Transkription des zweiten Nukleinsäuremoleküls exprimiert wird, zu vermitteln. Eine andere oder weitere Position im Genom kann bedeuten, dass das zweite Nukleotidmolekül außerhalb des chromosomalen Intervalls des Chromosom III von s3e4516s05 bis s3e5918s01 lokalisiert ist. Beispielsweise kann, sofern nicht schon im Ausganggenotyp vorhanden mittels Kreuzung oder Transformation das in der internationalen Anmeldung WO 2014/202044 A1 beschriebene RZ-3 Gen in die *wb*-R Pflanze der vorliegenden Pflanze eingebracht werden. In der vorliegenden Anmeldung werden daher *wb*-R/RZ-3-R Pflanzen offenbart, die vorzugsweise für eine und insbesondere bevorzugt für beide Resistenzgene homozygot sind. In diesem Zusammenhang versteht sich, dass aufgrund der Bereitstellung der Sequenzinformation für das *wb*-R Gen in der vorliegenden Anmeldung und bspw. die in der genannten WO 2014/202044 A1 beschriebenen Sequenzinformation für das RZ-3 Gen sich solche doppelt resistenten Pflanzen erstmals allein aufgrund Kreuzung und Marker-gestützter Selektion erhalten lassen, so dass es keiner gentechnischen Verfahren bedarf.

Dementsprechend betrifft die vorliegende Erfindung ebenfalls ein Verfahren zur Identifikation einer Pflanze der Gattung Beta, die resistent gegenüber dem Pathogen BNYVV ist, dadurch gekennzeichnet, dass das Verfahren den nachfolgenden Schritt umfasst:
(i) Nachweisen des Vorhandenseins des besagten Nukleinsäuremoleküls, wobei das davon kodierte Polypeptid (i) ein Glutamin (Q) an derjenigen Position aufweist, welche in der Referenz-Aminosäuresequenz SEQ ID NO: 2 der Position 307 entspricht, oder (ii) ein Arginin (R) an derjenigen Position aufweist, welche in der Referenz-Aminosäuresequenz SEQ ID NO: 2 der Position 437 entspricht, und/oder
   der Expression des besagten Nukleinsäuremoleküls, wobei das Nukleinsäuremolekül an denjenigen Stellen, die den Stellen in der Referenz-Nukleotidsequenz SEQ ID NO: 1 entsprechen, die folgenden Nukleotidsubstitutionen aufweist: (a) C statt A an Position 919, oder (b) G statt A an Position 1310; in der Pflanze bzw. einer Probe davon; und/oder
(ii) Nachweisen des Vorhandenseins des besagten Nukleinsäuremoleküls, wobei das davon kodierte Polypeptid (i) ein Glutamin (Q) an derjenigen Position aufweist, welche in der Referenz-Aminosäuresequenz SEQ ID NO: 2 der Position 307 entspricht, und (ii) ein Arginin (R) an derjenigen Position aufweist, welche in der Referenz-Aminosäuresequenz SEQ ID NO: 2 der Position 437 entspricht, und/oder
   der Expression des besagten Nukleinsäuremoleküls, wobei das Nukleinsäuremolekül an denjenigen Stellen, die den Stellen in der Referenz-Nukleotidsequenz SEQ ID NO: 1 entsprechen, die folgenden Nukleotidsubstitutionen aufweist: (a) C statt A an Position 919, und (b) G statt A an Position 1310; in der Pflanze bzw. einer Probe davon, und gegebenenfalls
(iii) Selektieren der BNYVV resistenten Pflanze.

Wie die vorliegende Anmeldung offenbart, kann das Verfahren vorteilhafterweise den Nachweis von mindestens einem Polymorphismus umfassen, der zu einem Aminosäureaustausch gegenüber der in Figur 1B (SEQ ID NO: 2) gezeigten Aminosäuresequenz führt, bevorzugt an einer der in der Abbildung 1D (SEQ ID NO: 4) gezeigten Aminosäuresequenz hervorgehobenen Stellen (siehe auch die Figurlegende zu Abbildung 1) mit den in Figur 1C (SEQ ID NO: 3) hervorgehobenen Polymorphismen unter Verwendung von molekularen Markern, welche die Polymorphismen, insbesondere diagnostische Polymorphismen, erkennen. Bevorzugt findet dieser Nachweis unter Verwendung von mindestens einem molekularen Marker pro Polymorphismus, insbesondere pro diagnostischen Polymorphismus, statt. Dem Fachmann ist bekannt, welche Markertechniken zum Nachweis eines entsprechenden Polymorphismus anzuwenden sind, und wie man molekulare Marker dafür konstruiert. Weiterhin offenbart die vorliegende Anmeldung molekulare Marker, welche einen Polymorphismus gemäß Figur 1C oder D beschreiben bzw. detektieren, wie auch die Verwendung eines molekularen Markers zur Detektion eines Polymorphismus gemäß Figur 1C und/oder D.

Weiterhin stellen die vorstehenden Identifikationsverfahren auch Verfahren zur Selektion einer Pflanze, welche eine Resistenz gegenüber BNYVV aufweist, dar. Das Verfahren zur Selektion umfasst einen abschließenden Schritt des Selektierens einer resistenten Pflanze.

Ferner können stromaufwärts zum *wb*-R Gen (wie SEQ ID NO: 3) angrenzende genomische DNA-Sequenzabschnitte und stromabwärts zum *wb*-R Gen angrenzende genomische DNA-Sequenzabschnitte, welche in unmittelbarer Umgebung, vorzugsweise auf Chromosom III lokalisiert und daher enggekoppelt mit dem *wb*-R Gen sind, als DNA-Regionen zur Entwicklung von diagnostischen Markern für *wb*-R verwendet werden.

Daher betrifft die vorliegende Erfindung ein Verfahren zur Selektion einer Pflanze, welche eine Resistenz gegenüber BNYVV aufweist.

Wie in der vorliegenden Anmeldung offenbart, kann das Verfahren zur Selektion die Verwendung eines molekularen Markers auf einer DNA-Sequenz gemäß SEQ ID NO: 3 und/oder auf einer DNA-Sequenz, die in unmittelbaren Umgebung lokalisiert sind, vorzugsweise auf Chromosom III, umfassen. Vorzugsweise liegen die in unmittelbaren Umgebung lokalisierten Marker gemäß den in den Beispielen beschrieben Markern s3e4516s05 und s3e5918s01 in einem Bereich von 0,11 cM, welche einer physikalischen Länge von etwa 100.000 bp entspricht von dem wb-R Gen und zeigen einen vergleichbaren diagnostischen Wert (DW) wie (a) s3e4516s05_cyt / DW=0,89; links flankierend und (b) s3e5918s01_ade / DW=0,91; rechts flankierend. Das in der vorliegenden Anmeldung beschriebene Verfahren umfasst ferner üblicherweise einen abschließenden Schritt des Selektierens einer resistenten Pflanze. Dem Fachmann ist bekannt, wie er auf Grundlage der offenbarten Sequenzinformationen Marker entwickelt und einsetzt.

Dementsprechend betrifft die vorliegende Erfindung auch ein Pflanze, vorteilhafterweise insbesondere BNYVV-resistente Pflanze oder einen Teil davon, welche durch ein wie vorstehend beschriebenes Verfahren identifiziert und gegebenenfalls selektiert wurde.

Insbesondere betrifft die vorliegende Erfindung eine Population von Pflanzen umfassend Pflanzen, die gemäß einem der vorstehend beschriebenen erfindungsgemäßen Verfahren erhältlich sind und vorzugsweise resistent gegen Wurzelbärtigkeit (Rizomania) bzw. BNYVV-Befall sind, und sich durch die Anwesenheit eines erfindungsgemäßen Nukleinsäuremoleküls auszeichnen. Vorzugsweise weist die Population mindestens 10, vorzugsweise 50, mehr bevorzugt 100, besonders bevorzugt 500 und insbesondere im landwirtschaftlichen Anbau bevorzugt mindestens 1000 Pflanzen auf. Vorzugsweise liegt der Anteil an Pflanzen in der Population, die nicht das erfindungsgemäße Nukleinsäuremolekül tragen und/oder anfällig für Wurzelbärtigkeit sind unter 25%, vorzugsweise unter 20%, mehr bevorzugt unter 15%, noch mehr bevorzugt 10%, und insbesondere bevorzugt unter 5%, 4%, 3%, 2%, 1% oder 0,5%, wenn überhaupt vorhanden.

Wie in der vorliegenden Anmeldung offenbart, können ferner folgende Vorteile für die Züchtung und die Entwicklung neuer resistenter Pflanzenlinien der Gattung Beta erzielt werden: Sequenzinformationen sowie die identifizierten Polymorphismen, welche eine Unterscheidung zwischen resistenten *wb*-R- und anfälligen *wb*-s-Allelen des offenbarten Gens erlauben, machen die Marker-Entwicklung direkt im Gen möglich, was insbesondere im Hinblick auf die Entwicklung von optimierten Elitelinien ohne "Linkage Drag" eine bedeutende Erleichterung für den Pflanzenzüchter darstellt. Außerdem kann die Kenntnis über die sequentielle Struktur zur Identifizierung von weiteren neuen Resistenzgenen, insbesondere gegen Rizomania, welche beispielsweise teilweise homolog oder ortholog sind, verwendet werden.

Die hier offenbarte Nutzung des resistenten Genallels in cis- oder trans-genetische Ansätzen eröffnet die Möglichkeit, neue resistente Sorten der Gattung Beta zu entwickeln, welche eine anhand des Dosiseffekts eine höhere Resistenz aufweisen oder in welchen durch das Stacking des offenbarten Gens mit anderen Resistenzgenen, insbesondere mit dem RZ-3 Gen ein Resistenzbruch vermieden werden und die Resistenzausprägung optimiert werden kann. Ferner sind Modifikationen des Gens mittels TILLING oder gezieltem Genome Engineering zur Entwicklung neuer Resistenzallele möglich.

Wie in der vorliegenden Anmeldung offenbart, kann das identifizierte resistente *wb*-R-Genallel in einem genetischen oder molekularen Stack mit anderen genetischen Elementen, welche agronomisch vorteilhafte Eigenschaften vermitteln können, in einer Pflanze verwendet werden. Hierdurch kann der ökonomische Wert von Kulturpflanzen deutlich gesteigert werden, indem beispielsweise die Ertragsleistung gesteigert wird oder neue Anbauflächen für eine Pflanze erschlossen werden, die zuvor unter anderem aufgrund biotischen Faktoren wie starkem Pathogendruck oder abiotischen Faktoren wie Trockenheit dem Anbau dieser Pflanze nicht zugänglich waren. Insbesondere betrifft die vorliegende Erfindung die Verwendung des identifizierten resistenten *wb*-R-Genallels in Verfahren zur Kontrolle des Befalls mit dem Pathogen Beet Necrotic Yellow Vein Virus (BNYVV) im agrarkulturellen oder hortikulturellen Anbau von Pflanzen der Gattung Beta, beispielsweise umfassend das Identifizieren und Selektieren von Pflanzen der Gattung Beta mit Hilfe eines der vorstehend beschriebenen Verfahren Anbauen der so ausgewählten Pflanzen oder Nachkommen davon.

Eine agronomisch vorteilhafte Eigenschaft ist beispielsweise eine Toleranz gegenüber einem Herbizid wie Glyphosat, Glufosinat oder ALS-Inhibitoren. Dem Fachmann sind aus dem Stand der Technik zahlreiche weitere Herbizide und deren Anwendbarkeit bekannt. Er kann auf den Stand der Technik zurückgreifen, um Kenntnis zu erlangen, welche genetischen Elemente in welcher Art und Weise zu verwenden sind, um eine entsprechende Toleranz in Pflanzen zu implementieren. Ein weiteres Beispiel für eine agronomisch vorteilhafte Eigenschaft ist eine zusätzliche Pathogenresistenz, wobei Pathogene beispielsweise Insekten, Viren, Nematoden, Bakterien oder Fungi sein können. Durch Kombination unterschiedlicher Pathogenresistenzen/-toleranzen kann beispielsweise eine breite Pathogenabwehr für eine Pflanze erreicht werden, da genetische Elemente untereinander ergänzende Wirkungen aufweisen können. Hierfür sind dem Fachmann als genetische Elemente beispielsweise zahlreiche Resistenzgene bekannt. Ein weiteres Beispiel für eine agronomisch vorteilhafte Eigenschaft ist eine Kühle- oder Frosttoleranz. Pflanzen, welche diese Eigenschaft aufweisen, könnten früher im Jahr bereits ausgesät werden oder könnten länger zum Beispiel auch über Frostperioden im Feld verbleiben, was beispielsweise zu gesteigerten Erträgen führen kann. Auch hier kann der Fachmann auf den Stand der Technik zurückgreifen, um geeignete genetische Elemente zu finden. Weitere Beispiele für agronomisch vorteilhafte Eigenschaften sind Wassernutzungseffizienz, Stickstoffnutzungseffizienz sowie Ertrag. Genetische Elemente, welche eingesetzt werden können, um solche Eigenschaften zu vermitteln, könnten im Stand der Technik gefunden werden.

Dem Fachmann sind weiterhin zahlreichen Modifikationen zur Pathogenabwehr bekannt. Neben den häufig beschriebenen Familien der R-Gene könnten der Avr/R-Ansatz, die Avr-Gen-Komplementierung (WO 2013/127379), die Autoaktivierung eines R-Gens (WO 2006/128444), der HIGS (host induced gene silencing)-Ansatz (z.B. WO2013/050024) oder der VIGS (virus induced gene silencing)-Ansatz vorteilhaft eingesetzt werden. Insbesondere die Autoaktivierung eines R-Gens könnte für die vorliegende Erfindung von Bedeutung sein. Hierfür ist eine Nukleinsäure zu erstellen, die für ein autoaktiviertes Resistenzprotein zur Erzeugung einer Resistenz gegenüber Pathogenen bei Pflanzen kodiert. Diese Nukleinsäure weist dann nur einen begrenzten Teil eines NBS-LRR-Resistenzgens wie das *wb*-R-Gen aufweist, der sich vom 5'-Ende des kodierenden Bereichs des NBS-LRR-Resistenzgens stromabwärts bis zum Beginn der NBS-Domäne des NBS-LRR-Resistenzgens erstreckt, wobei das NBS-LRR-Resistenzgen kein TIR-NBS-LRR-Resistenzgen ist.

Ferner offenbart die vorliegende Anmeldung auch die Verwendung des resistenten *wb*-R-Genallels*,* identifiziert mit einem oben beschriebenen Verfahren, zur Kombination mit einer vorstehenden Modifikationen oder mit einem vorstehend beschriebenen genetischen Element, welches eine oder mehrere agronomisch vorteilhafte Eigenschaften in einer Pflanze vermitteln kann.

Ferner offenbart die vorliegende Anmeldung neben der erfindungsgemäßen Pflanze auch Samen oder Nachkommen, oder Organe, Pflanzenteile, Gewebe oder Zellen davon in der Herstellung von üblicherweise aus nachwachsenden Rohstoffen gefertigten Produkten wie Nahrungsmittel und Futtermittel, vorzugsweise Zucker oder Sirup (Melasse), wobei die Melasse auch für industrielle Anwendungen genutzt wird, beispielsweise in der Alkoholgewinnung oder als Nährmedium für die Herstellung von biotechnologischen Produkten, in der Herstellung von Werkstoffen oder Stoffen für die chemischen Industrie, z.B. Feinchemikalien, Arzneimitteln bzw. Vorstufen davon, Diagnostika, Kosmetika, Bioethanol oder Biogas. Ein Beispiel für die Verwendung von Zuckerrübe als biogener Rohstoff in Biogasanlagen ist in der Anmeldung DE 10 2012 022 178 A1 beschrieben, siehe z.B. Absatz 10.

Die folgenden Beispiele erläutern die Erfindung. Sofern nicht anders angegeben, wurden molekularbiologischen Standardmethoden verwendet, siehe beispielsweise (Sambrook et al., Molecular Cloning: A Laboratory Manual 3rd Ed. Cold Spring Harbor Laboratory Press, Cold Spring Harbor, NY, 2001), Fritsch *et al.*, Cold Spring Harbor Laboratory Press: 1989; Mayer et al., Immunochemical Methods In Cell And Molecular Biology, eds., Academic Press, London, 1987) und Weir et al., Handbook Of Experimental Immunology, Volumes I-IV, Blackwell, eds., 1986).

### BEISPIELE

### Beispiel 1: Identifizierung eines gegen Rizomania (Wurzelbärtigkeit, WB) resistenzvermittelnden Gens (WB1) und dazugehöriger sensitiven Allele

In einer Population von Zuckerrübe (Beta vulgaris L.) mit einer Introgression aus einer Akzession von *Beta vulgaris subsp. maritima* konnte in dieser Introgression ein gegen Wurzelbärtigkeit (Rizomania) resistenzvermittelndes Gen bzw. Genomsegment mittels Markern mit gutem diagnostischen Wert (DW) nachgewiesen werden, (a) s3e4516s05_cyt / DW=0,89; links flankierend und (b) s3e5918s01_ade / DW=0,91; rechts flankierend. Die beiden Marker sind allerdings nicht volldiagnostisch wegen des Auftretens von Nullallelen in unterschiedlichen Pedigrees. Die genomische Region zwischen s3e4516s05 und s3e5918s01 umfasst eine genetische Länge von 0,11 cM, welche einer physikalischen Länge von etwa 100.000 bp entspricht. Diese Sequenzregion ist hoch-repetitiv und zeigt eine starke strukturelle Variation in unterschiedlichen Genotypen; es ist daher sehr schwierig weitere diagnostische Marker zu entwickeln. Durch die Unkenntnis der genetischen Struktur des Rizomaniaresistenzvermittelnden Genomsegment ist es zudem nur beschränkt möglich, den potenziellen "negativen Linkage Drag" um das kausale Gen herum weiter zu reduzieren.

Erste Experimente zur näheren Eingrenzung des relevanten Genomsegments und ggf. Auffinden eines die beobachtete Resistenz vermittelnden Gens bzw. eines für die Rizomania sensitive Eigenschaft der Pflanzen verantwortlichen Genlocus schlugen fehl, u.a. da die Zielregion sehr repetitiv ist und eine starke strukturelle Variation (Nullallele) in vielen Genotypen zeigt, und weitere Marker mit hohen diagnostischen Wert nicht zur Verfügung standen. Außerdem lieferte die Expressionsanalyse von Kandidatengenen in der Zielregion zunächst keine Evidenz, d.h. spezifische Antwort auf die Rhizomania-Infektion. Schließlich gestaltete sich auch die Phänotypisierung der Pflanzen schwierig, da die Resistenzausprägung aus unbekannten Gründen nicht immer eindeutig war, so dass zunächst auch das Vorhandensein einer multigenen Resistenz vermutet wurde und/oder epigenetische Effekte, die erst durch Erhöhung der Anzahl der untersuchten Pflanzen mit 90-180 Nachkommen und mit intensiven statistischen Verfahren (t-Test, Poweranalyse) ausgeschossen werden konnten.

In weiteren durchgeführten Experimenten und Analysen konnte dann mittels "Map Based Cloning", das u.a. die Schritte genetische Feinkartierung, physikalische Kartierung, WHG (Ganzgenom) Sequenzanalyse, Aufbau einer sehr großen spaltenden Population von über 2000 F2-Nachkommen, Rekombinanten-Screen, Markerentwicklung in der Zielregion, vergleichende BAC Sequenzierung in resistenten (RR) und sensitiven/anfälligen (ss) Genotypen, bioinformatische Analysen, Proteinvorhersagen, und Vergleich der Proteine umfasste, festgestellt werden, dass ein NB-ARC (NBS-LRR)-Gen für die beobachtete Rizomania-Resistenz verantwortlich ist. Dieses NBS-LRR-Gen wurde durch intensive Feinkartierung identifiziert, wobei sich wegen der Sequenzkomplexität die Assemblierung der RR- und ss-Sequenzen nicht ohne Weiteres ergaben, sondern erst durch die Analyse der Nachkommenschaft von den 4 engsten rekombinanten Pflanzen (2 direkte Rekombinanten links und zwei direkte Rekombinanten rechts um das Gen herum) das NBS-LRR-Gen punktgenau identifiziert werden konnte. Die Sequenz des NBS-LRR-Gens des resistenten Genotyp ist in SEQ ID NO: 3 wiedergegeben und das dazugehörige Gen bzw. Genotyp "wb-R" für "Wurzelbärtigkeit-Resistenz" genannt. In dem NBS-LRR-Gen wurden insgesamt 17 "nicht-synonyme" Einzelnukleotid-Polymorphismen (SNPs) gefunden (Polymorphismen, die zu einem Aminosäureaustausch in dem Protein führen). Basierend auf Sequenzdaten bestehend aus sensitiven und resistenten Genotypen zeigten sich davon 5 Aminosäureaustausche als volldiagnostisch:
**K307Q,** "C" statt "A" in der genomischen Sequenz an Position 919 im resistenten Genotyp, was das kodierte Lysin (K) an Position 307 des anfälligen Gens durch ein Glutamin (Q) ersetzt,
**Q437R,** "G" statt "A" in der genomischen Sequenz an Position 1310 im resistenten Genotyp, was das kodierte Glutamin (Q) an Position 437 des anfälligen Gens durch ein Arginin (R) ersetzt,
**R566H,** "A" statt "G" in der genomischen Sequenz an Position 1697 im resistenten Genotyp, was das kodierte Arginin (R) an Position 566 des anfälligen Gens durch ein Histidin (H) ersetzt,
**Q731K,** "A" statt "C" in der genomischen Sequenz an Position 2191 im resistenten Genotyp, was das kodierte Glutamin (Q) an Position 731 des anfälligen Gens durch ein Lysin (K) ersetzt, und
**P831S,** "T" statt "C" in der genomischen Sequenz an Position 2491 im resistenten Genotyp, was das kodierte Prolin (P) an Position 831 des anfälligen Gens durch ein Serin (S) ersetzt; siehe Abbildung 1. Aufgrund der Rekombinationspunkte kann einer von den beiden oder beide Aminosäureaustausche K307Q oder Q437R als kausal für die Resistenzvermittlung betrachtet werden. Die entsprechenden Gensequenzen bzw. Genotypen, die mit dem für Wurzelbärtigkeit sensitiven Phänotyp korrelieren werden - auch *"wb*-s*"* für "Wurzelbärtigkeit-sensitiv" genannt.

### Beispiel 2: Validierung des wb-R Gens mittels RNAi-Ansatz

Neben der oben beschriebenen Verifizierung des Gens mittels engen Rekombinanten kann als ein weiterer Nachweis die Resistenzwirkung des Gens mittels RNA-Interferenz gezeigt werden; siehe bspw. die in den Beispielen (Examples) der internationalen Anmeldung WO 2014/202044 A1 beschriebene Verifizierung des RZ-3 Gens oder die in den Beispielen (Examples) der internationalen Anmeldung WO 2011/032537 A1 beschriebene Verifizierung des vil Gens. Hierfür wird ein resistenter Standard-Zuckerrübengenotyp mit einem DNA-Konstrukt transformiert, welches eine doppelsträngige hairpin-RNA kodiert. Diese dsRNA ist in der Lage post-transkriptional ein Gene Silencing zu bewirken, welches das resistente wb-R Genallel in seiner Wirkung vermindern oder ausschalten würde, wodurch der zuvor resistente Zuckerrübengenotyp sensitiv gegenüber Wurzelbärtigkeit (Rizomania) werden sollte.

Zur Bereitstellung eines geeigneten DNA-Konstrukts wird ein definierter Zielsequenzbereich des resistenten wb-R Genallels von beispielsweise 400-500 Basenpaaren Länge vorzugweise aus einer Region der kodierenden Sequenz ausgewählt, welche spezifisch für das resistente wb-R Genallels sind, durch PCR amplifiziert und sowohl in sense- als auch in antisense-Richtung in den Vektor pZFN kloniert, der zur Synthese von hairpin-Strukturen geeignet ist (siehe Fig. 6 der internationalen Anmeldung WO 2014/202044 A1). Dieser Vektor weist einen doppelten CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen nos-Terminator auf. Die Transformation der Zuckerrüben mit dem bereitgestellten Vektor erfolgt nach dem Protokoll von Lindsey & Gallois, J. Exp. Bot. 41 (1990), 529-536, unter Verwendung des Antibiotikums Kanamycin als Selektionsmarker. Nach mehreren Selektionsschritten wird die erfolgreiche Transformation an transgenen Sprosse über PCR mittels Nachweis der Anwesenheit des nptII-Gen, des AAP6-Intron und der beiden t-DNA-Bordersequenzen (LB/RB) und der Abwesenheit von *vir* überprüft. Positive Sprosse werden *in-vitro* auf jeweils 30 Sprosse klonal vermehrt, bewurzelt und im Gewächshaus in Erde überführt. Etwa 2 Wochen später werden die transgenen Zuckerrübenpflanzen in Rizomania-kontaminierte Erde pikiert, in welcher sie für 8 bis 10 Wochen kultiviert werden. Als Kontrolle werden unter denselben Bedingungen nicht transformierte Pflanzen desselben resistenten genetischen Standard-Transformationshintergrunds herangezogen. Zum Nachweis der Ausprägung der Rizomania werden die Wurzeln der Zuckerrübenpflanzen geerntet und mittels ELISA-Test der BNYVV-Befall quantifiziert, wobei ein niedriger ELISA-Wert eine Resistenz und ein hoher Wert eine Sensitivität anzeigt (Mechelke 1997, oben; Clark & Adams, J. Gen. Virol. 34 (1977), 475-483). Der ELISA-Wert der transformierten Zuckerrüben ist erwartungsgemäß mit einem Mittelwert von 3-4 signifikant höher als der ELISA-Wert der weiterhin resistenten Kontrolle mit einem Mittelwert von 1-2 und vergleichbar mit dem sensitiven Standard. Danach kann durch die Ergebnisse des ELISA-Tests gezeigt werden, dass durch das spezifische Gene-Silencing des resistenten *wb*-R-Allels im Transformationshintergrund eine zuvor resistente Pflanze sensitiv gegenüber BNYVV wird. Folglich kann das *wb*-R Gen der vorliegenden Erfindung eindeutig als das Resistenzgen verifiziert werden.

Die Validierung der Genfunktion kann ferner durch Komplementation einer WB sensitiven Pflanze, insbesondere Zuckerrübe mit einem erfindungsgemäßen *wb*-R Gen durchgeführt werden, beispielsweise in dem in einen sensitiven Genotyp ein Pflanzenexpressionsvektor transformiert wird, der ein Nukleinsäuremolekül enthält mit der Nukelotidsequenz der SEQ ID NO: 3 bzw. einer äquivalenten Nukelotidsequenz, die ein Polypeptid mit einer Aminosäuresequenz der SEQ ID NO: 4 kodiert, jeweils unter der Kontrolle eines konstitutiven Promotor. Für die Transformation kann grundsätzlich auf den vorstehend beschriebenen pZFN-Vektor und die genannten Techniken zurückgegriffen werden, oder auch auf diejenige, die vorstehend in der allgemeinen Beschreibung beschrieben sind.

## Patentansprüche

1. Nukleinsäuremolekül, welches ein Polypeptid kodiert, das in der Lage ist, eine Resistenz gegenüber einem Pathogen in einer Pflanze, in welcher das Polypeptid exprimiert wird, zu vermitteln, **dadurch gekennzeichnet, dass** das Nukleinsäuremolekül eine Nukleotidsequenz umfasst, welche ausgewählt ist aus
(a) einer Nukleotidsequenz, die ein Polypeptid mit einer Aminosäuresequenz, die zu mindestens 80 % identisch mit der SEQ ID NO: 2 ist, kodiert,
wobei das Nukleinsäuremolekül eine oder mehrere Mutationen aufweist, welche dazu führen, dass das Polypeptid, das durch das Nukleinsäuremolekül kodiert wird, an derjenigen Position, welche in der Referenz-Aminosäuresequenz SEQ ID NO: 2 der Position 307 entspricht, eine Aminosäure aufweist, welche sich von Lysin (K) unterscheidet, und/oder an derjenigen Position, welche in der Referenz-Aminosäuresequenz SEQ ID NO: 2 der Position 437 entspricht, eine Aminosäure aufweist, welche sich von Glutamin (Q) unterscheidet; und
(b) einer Nukleotidsequenz, mit der komplementären Sequenz zu SEQ ID NO: 1 unter stringenten Bedingungen hybridisiert, wobei die Nukleotidsequenz an denjenigen Stellen, die den Stellen in der Referenz-Nukleotidsequenz SEQ ID NO: 1 entsprechen, eine oder mehrere der folgenden Nukleotidsubstitutionen aufweist: C statt A an Position 919; G statt A an Position 1310; A statt G an Position 1697; A statt C an Position 2191; und/oder T statt C an Position 2491;
vorzugsweise wobei das Pathogen das Beet necrotic yellow vein virus (BNYVV) und die Pflanze eine Pflanze der Gattung Beta, vorzugsweise Zuckerrübe (Beta vulgaris L.), ist.

2. Nukleinsäuremolekül nach Anspruch 1, **dadurch gekennzeichnet, dass** das kodierte Polypeptid weiterhin (i) an derjenigen Position, welche in der Referenz-Aminosäuresequenz SEQ ID NO: 2 der Position 566 entspricht, eine Aminosäure aufweist, welche sich von Arginin (R) unterscheidet, und/oder (ii) an derjenigen Position, welche in der Referenz-Aminosäuresequenz SEQ ID NO: 2 der Position 731 entspricht, eine Aminosäure aufweist, welche sich von Glutamin (Q) unterscheidet, und/oder (iii) an derjenigen Position, welche in der Referenz-Aminosäuresequenz SEQ ID NO: 2 der Position 831 entspricht, eine Aminosäure aufweist, welche sich von Prolin (P) unterscheidet.

3. Nukleinsäuremolekül nach Anspruch 1 oder 2, **dadurch gekennzeichnet, dass** das kodierte Polypeptid (i) ein Glutamin (Q) an derjenigen Position aufweist, welche in der Referenz-Aminosäuresequenz SEQ ID NO: 2 der Position 307 entspricht, und/oder (ii) ein Arginin (R) an derjenigen Position aufweist, welche in der Referenz-Aminosäuresequenz SEQ ID NO: 2 der Position 437 entspricht, und/oder (iii) ein Histidin (H) an derjenigen Position aufweist, welche in der Referenz-Aminosäuresequenz SEQ ID NO: 2 der Position 566 entspricht, und/oder (iv) ein Lysin (K) an derjenigen Position aufweist, welche in der Referenz-Aminosäuresequenz SEQ ID NO: 2 der Position 731 entspricht, und/oder (v) ein Serin (S) an derjenigen Position aufweist, welche in der Referenz-Aminosäuresequenz SEQ ID NO: 2 der Position 831 entspricht.

4. Nukleinsäuremolekül nach Anspruch 3, **dadurch gekennzeichnet, dass** das Nukleinsäuremolekül an denjenigen Stellen, die den Stellen in der Referenz-Nukleotidsequenz SEQ ID NO: 1 entsprechen, eine oder mehrere der folgenden Nukleotidsubstitutionen aufweist:
(a) C statt A an Position 919;
(b) G statt A an Position 1310;
(c) A statt G an Position 1697;
(d) A statt C an Position 2191; und/oder
(e) T statt C an Position 2491.

5. Nukleinsäuremolekül nach einem der Ansprüche 1 bis 4, **dadurch gekennzeichnet, dass** das Nukleinsäuremolekül ein Polypeptid mit einer Aminosäuresequenz gemäß SEQ ID NO: 4 kodiert und/oder die kodierende DNA-Sequenz gemäß SEQ ID NO: 3 umfasst.

6. Vektor, welcher das Nukleinsäuremolekül nach einem der Ansprüche 1 bis 5 umfasst.

7. Transgene Pflanzenzelle, vorzugsweise von einer Pflanze der Gattung Beta, welche das Nukleinsäuremolekül nach einem der Ansprüche 1 bis 5 als Transgen, optional unter der Kontrolle eines heterologen Promoters, oder den Vektor nach Anspruch 6 umfasst.

8. Transgene Pflanze, vorzugsweise der Gattung Beta, oder ein Teil davon, die eine Pflanzenzelle nach Anspruch 7 umfasst.

9. BNYVV-resistente Pflanze der Species Beta vulgaris oder ein Teil davon, in welcher das Nukleinsäuremolekül nach einem der Ansprüche 1 bis 5 endogen vorhanden ist, oder in welcher die eine oder mehrere Mutationen, definiert in einem der Ansprüche 1 bis 4, in ein endogenes Nukleinsäuremolekül mit einer Nukleotidsequenz, die die Sequenz gemäß SEQ ID NO: 1 umfasst oder die mit der komplementären Sequenz zu SEQ ID NO: 1 unter stringenten Bedingungen hybridisiert, eingebracht worden sind, wobei die Pflanze oder ein Teil davon nicht zur Subspezies *Beta vulgaris subsp. maritima* gehört.

10. Pflanze nach Anspruch 8 oder 9, **dadurch gekennzeichnet, dass** die Pflanze eine Hybridpflanze oder doppelhaploide Pflanze ist und/oder dass das Nukleinsäuremolekül oder die eine oder mehrere Mutationen heterozygot oder homozygot vorliegen.

11. Samen von der Pflanze nach einem der Ansprüche 8 bis 10, wobei der Samen ein oder mehrere der vorgenannten Nukleinsäuremoleküle oder Vektoren transgen oder endogen umfasst.

12. Verfahren zur Herstellung einer BNYVV-resistenten Pflanze, umfassend die folgenden Schritte:
(a) Mutagenisieren von Pflanzenzellen und anschließendem Regenerieren von Pflanzen aus den mutagenisierten Pflanzenzellen oder Mutagenisieren von Pflanzen, und
(b) Identifizieren einer Pflanze aus (a), welche in einem endogenen Nukleinsäuremolekül die eine oder mehrere Mutationen definiert in einem der Ansprüche 1 bis 5 aufweist.

13. Verfahren zur Identifikation einer Pflanze der Gattung Beta, die resistent gegenüber dem Pathogen BNYVV ist, **dadurch gekennzeichnet, dass** das Verfahren den nachfolgenden Schritt umfasst:
(i) Nachweisen des Vorhandenseins eines Nukleinsäuremoleküls gemäß Anspruch 3, wobei das davon kodierte Polypeptid (i) ein Glutamin (Q) an derjenigen Position aufweist, welche in der Referenz-Aminosäuresequenz SEQ ID NO: 2 der Position 307 entspricht, oder (ii) ein Arginin (R) an derjenigen Position aufweist, welche in der Referenz-Aminosäuresequenz SEQ ID NO: 2 der Position 437 entspricht, und/oder der Expression des Nukleinsäuremoleküls gemäß Anspruch 4, wobei das Nukleinsäuremolekül an denjenigen Stellen, die den Stellen in der Referenz-Nukleotidsequenz SEQ ID NO: 1 entsprechen, die folgenden Nukleotidsubstitutionen aufweist: (a) C statt A an Position 919, oder (b) G statt A an Position 1310; in der Pflanze bzw. einer Probe davon; und/oder
(ii) Nachweisen des Vorhandenseins eines Nukleinsäuremoleküls gemäß Anspruch 3, wobei das davon kodierte Polypeptid (i) ein Glutamin (Q) an derjenigen Position aufweist, welche in der Referenz-Aminosäuresequenz SEQ ID NO: 2 der Position 307 entspricht, und (ii) ein Arginin (R) an derjenigen Position aufweist, welche in der Referenz-Aminosäuresequenz SEQ ID NO: 2 der Position 437 entspricht, und/oder der Expression des Nukleinsäuremoleküls gemäß Anspruch 4, wobei das Nukleinsäuremolekül an denjenigen Stellen, die den Stellen in der Referenz-Nukleotidsequenz SEQ ID NO: 1 entsprechen, die folgenden Nukleotidsubstitutionen aufweist: (a) C statt A an Position 919, und (b) G statt A an Position 1310; in der Pflanze bzw. einer Probe davon, und gegebenenfalls
(iii) Selektieren der BNYVV resistenten Pflanze.

14. Population von Pflanzen umfassend Pflanzen nach einem der Ansprüche 9 oder 10 oder Pflanzen, welche durch ein Verfahren gemäß Anspruch 13 identifiziert und gegebenenfalls selektiert wurde.

15. Verfahren zur Kontrolle des Befalls mit dem Pathogen Beet Necrotic Yellow Vein Virus (BNYVV) im agrarkulturellen oder hortikulturellen Anbau von Pflanzen der Gattung Beta, umfassend
I) das Identifizieren und Selektieren von Pflanzen der Gattung Beta mit Hilfe eines Verfahren nach Anspruch 13 und
II) Anbauen der Pflanzen aus I) oder Nachkommen davon.

## Claims

1. Nucleic acid molecule encoding a polypeptide capable of mediating a resistance to a pathogen in a plant in which the polypeptide is expressed, **characterized in that** the nucleic acid molecule comprises a nucleotide sequence selected from
(a) a nucleotide sequence encoding a polypeptide with an amino acid sequence that is at least 80% identical to the SEQ ID NO: 2,
wherein the nucleic acid molecule comprises one or more mutations that cause that the polypeptide, encoded by the nucleic acid molecule, has an amino acid different from lysine (K) at the position corresponding to position 307 in the reference amino acid sequence SEQ ID NO: 2, and/or at the position corresponding to position 437 in the reference amino acid sequence SEQ ID NO: 2, comprises an amino acid distinct from glutamine (Q); and
(b) a nucleotide sequence that hybridizes with the complementary sequence to SEQ ID NO: 1 under stringent conditions, wherein the nucleotide sequence has one or more of the following nucleotide substitutions at those sites corresponding to the sites in the reference nucleotide sequence SEQ ID NO: 1: C instead of A at position 919; G instead of A at position 1310; A instead of G at position 1697; A instead of C at position 2191; and/or T instead of C at position 2491;
preferably wherein the pathogen is the beet necrotic yellow vein virus (BNYVV) and the plant is a plant of the genus Beta, preferably sugar beet (*Beta vulgaris L*.).

2. The nucleic acid molecule according to claim 1, **characterized in that** the encoded polypeptide further (i) has an amino acid different from arginine (R) at the position corresponding to position 566 in the reference amino acid sequence SEQ ID NO: 2, and/or (ii) at the position corresponding to position 731 in the reference amino acid sequence SEQ ID NO: 2, which is distinct from glutamine (Q), and/or (iii) at the position corresponding to position 831 in the reference amino acid sequence SEQ ID NO: 2, has an amino acid distinct from proline (P).

3. The nucleic acid molecule according to claim 1 or 2, **characterized in that** the encoded polypeptide (i) has a glutamine (Q) at the position corresponding to position 307 in the reference amino acid sequence SEQ ID NO: 2, and/or (ii) has an arginine (R) at the position corresponding to position 437 in the reference amino acid sequence SEQ ID NO: 2, and/or (iii) has a histidine (H) at that position, which corresponds to position 566 in the reference amino acid sequence SEQ ID NO: 2, and/or (iv) has a lysine (K) at the position corresponding to position 731 in the reference amino acid sequence SEQ ID NO: 2, and/or (v) has a serine (S) at that position corresponding to position 831 in the reference amino acid sequence SEQ ID NO: 2.

4. The nucleic acid molecule according to claim 3, **characterized in that** the nucleic acid molecule has one or more of the following nucleotide substitutions at those sites corresponding to the sites in the reference nucleotide sequence SEQ ID NO: 1:
(a) C instead of A at position 919;
(b) G instead of A at position 1310;
(c) A instead of G at position 1697;
(d) A instead of C at position 2191; and/or
(e) T instead of C at position 2491.

5. The nucleic acid molecule according to any one of claims 1 to 4, **characterized in that** the nucleic acid molecule encodes a polypeptide with an amino acid sequence according to SEQ ID NO: 4 and/or comprises the coding DNA sequence according to SEQ ID NO: 3.

6. A vector comprising the nucleic acid molecule according to any one of claims 1 to 5.

7. A transgenic plant cell, preferably from a plant of the genus Beta, comprising the nucleic acid molecule according to any one of claims 1 to 5 as a transgene, optionally under the control of a heterologous promoter, or the vector according to claim 6.

8. A transgenic plant, preferably of the genus Beta, or a part thereof, comprising a plant cell according to claim 7.

9. A BNYVV-resistant plant of the species *Beta vulgaris* or a part thereof in which the nucleic acid molecule is endogenously present according to any one of claims 1 to 5, or in which one or more mutations, defined in any one of claims 1 to 4, into an endogenous nucleic acid molecule having a nucleotide sequence comprising the sequence according to SEQ ID NO: 1, or which is associated with the complementary sequence to SEQ ID NO: 1 which have been hybridised under stringent conditions, whereby the plant or part of it does not belong to the subspecies *Beta vulgaris subsp. maritima.*

10. The plant according to claim 8 or 9, **characterized in that** the plant is a hybrid plant or a double haploid plant and/or that the nucleic acid molecule or one or more mutations are heterozygous or homozygous.

11. A seed from the plant according to any one of claims 8 to 10, wherein the seed comprises one or more of the aforementioned nucleic acid molecules or vectors transgenically or endogenously.

12. Method for producing a BNYVV-resistant plant, comprising the following steps:
(a) Mutagenization of plant cells and subsequent regeneration of plants from the mutagenized plant cells or mutagenization of plants, and
(b) Identification of a plant from (a) having one or more mutations in an endogenous nucleic acid molecule in any one of the claims 1 to 5.

13. Method for identifying a plant of the genus Beta which is resistant to the pathogen BNYVV, **characterized in that** the method comprises the following step:
(i) Evidence of the presence of a nucleic acid molecule according to claim 3, wherein the polypeptide encoded thereof (i) has a glutamine (Q) at the position corresponding to position 307 in the reference amino acid sequence SEQ ID NO: 2, or (ii) has an arginine (R) at the position corresponding to position 437 in the reference amino acid sequence SEQ ID NO: 2, and/or
the expression of the nucleic acid molecule according to claim 4, wherein the nucleic acid molecule has the following nucleotide substitutions at those sites corresponding to the sites in the reference nucleotide sequence SEQ ID NO: 1: (a) C instead of A at position 919, or (b) G instead of A at position 1310; in the plant or a sample thereof; and/or
(ii) Evidence of the presence of a nucleic acid molecule according to claim 3, wherein the polypeptide encoded therein (i) has a glutamine (Q) at the position corresponding to position 307 in the reference amino acid sequence SEQ ID NO: 2, and (ii) has an arginine (R) at the position corresponding to position 437 in the reference amino acid sequence SEQ ID NO: 2, and/or
the expression of the nucleic acid molecule according to claim 4, wherein the nucleic acid molecule has the following nucleotide substitutions at those sites corresponding to the sites in the reference nucleotide sequence SEQ ID NO: 1: (a) C instead of A at position 919, and (b) G instead of A at position 1310; in the plant or a sample of it, and, if necessary,
(iii) Selection of the BNYVV resistant plant.

14. A population of plants comprising plants according to any one of claims 9 or 10 or plants identified and, if necessary, selected by a method according to claim 13.

15. Methods for the control of infestation with the pathogen Beet Necrotic Yellow Vein Virus (BNYVV) in the agricultural or horticultural cultivation of plants of the genus Beta, comprising
(I) the identification and selection of plants of the genus Beta by means of a method according to claim 13, and
II) Cultivation of the plants from I) or descendants thereof.

## Revendications

1. Molécule d'acide nucléique, qui code pour un polypeptide qui est en mesure de promouvoir une résistance à un pathogène dans une plante, dans laquelle le polypeptide est exprimé, **caractérisée en ce que** la molécule d'acide nucléique comprend une séquence nucléotidique qui est choisie parmi
(a) une séquence nucléotidique qui code pour un polypeptide présentant une séquence d'acides aminés qui est identique à raison d'au moins 80% à la SEQ ID NO : 2,
la molécule d'acide nucléique présentant une ou plusieurs mutations qui font que le polypeptide qui est codé par la molécule d'acide nucléique présente, dans la position qui correspond à la position 307 dans la séquence d'acides aminés de référence SEQ ID NO : 2, un acide aminé qui se distingue de la lysine (K) et/ou, dans la position qui correspond à la position 437 dans la séquence d'acides aminés de référence SEQ ID NO : 2, un acide aminé qui se distingue de la glutamine (Q) ; et
(b) une séquence nucléotidique qui s'hybride dans des conditions stringentes avec la séquence complémentaire à la séquence SEQ ID NO : 1, la séquence nucléotidique présentant, dans les sites qui correspondent aux sites dans la séquence nucléotidique de référence SEQ ID NO : 1, une ou plusieurs des substitutions nucléotidiques suivantes : C au lieu de A dans la position 919 ; G au lieu de A dans la position 1310 ; A au lieu de G dans la position 1697 ; A au lieu de C dans la position 2191 ; et/ou T au lieu de C dans la position 2491 ;
le pathogène étant de préférence le virus des nervures jaunes nécrotiques de la betterave (BNYVV) et la plante étant une plante du genre Beta, de préférence la betterave sucrière (Beta vulgaris L.).

2. Molécule d'acide nucléique selon la revendication 1, **caractérisée en ce que** le polypeptide codé présente en outre (i) dans la position qui correspond à la position 566 dans la séquence d'acides aminés de référence SEQ ID NO : 2, un acide aminé qui se distingue de l'arginine (R) et/ou (ii) dans la position qui correspond à la position 731 dans la séquence d'acides aminés de référence SEQ ID NO : 2, un acide aminé qui se distingue de la glutamine (Q) et/ou (iii) dans la position qui correspond à la position 831 dans la séquence d'acides aminés de référence SEQ ID NO : 2, un acide aminé qui se distingue de la proline (P).

3. Molécule d'acide nucléique selon la revendication 1 ou 2, **caractérisée en ce que** le polypeptide codé présente (i) une glutamine (Q) dans la position qui correspond à la position 307 dans la séquence d'acides aminés de référence SEQ ID NO : 2 et/ou (ii) une arginine (R) dans la position qui correspond à la position 437 dans la séquence d'acides aminés de référence SEQ ID NO : 2 et/ou (iii) une histidine (H) dans la position qui correspond à la position 566 dans la séquence d'acides aminés de référence SEQ ID NO : 2 et/ou (iv) une lysine (K) dans la position qui correspond à la position 731 dans la séquence d'acides aminés de référence SEQ ID NO : 2 et/ou (v) une sérine (S) dans la position qui correspond à la position 831 dans la séquence d'acides aminés de référence SEQ ID NO : 2.

4. Molécule d'acide nucléique selon la revendication 3, **caractérisée en ce que** la molécule d'acide nucléique présente, dans les sites qui correspondent aux sites dans la séquence nucléotidique de référence SEQ ID NO : 1, une ou plusieurs des substitutions nucléotidiques suivantes :
(a) C au lieu de A dans la position 919 ;
(b) G au lieu de A dans la position 1310 ;
(c) A au lieu de G dans la position 1697 ;
(d) A au lieu de C dans la position 2191 ; et/ou
(e) T au lieu de C dans la position 2491.

5. Molécule d'acide nucléique selon l'une des revendications 1 à 4, **caractérisée en ce que** la molécule d'acide nucléique code pour un polypeptide présentant une séquence d'acides aminés selon la séquence SEQ ID NO : 4 et/ou comprend la séquence codante d'ADN selon la séquence SEQ ID NO : 3.

6. Vecteur qui comprend la molécule d'acide nucléique selon l'une des revendications 1 à 5.

7. Cellule végétale transgénique, de préférence d'une plante du genre Beta, qui comprend la molécule d'acide nucléique selon l'une des revendications 1 à 5 en tant que transgène, éventuellement sous le contrôle d'un promoteur hétérologue, ou le vecteur selon la revendication 6.

8. Plante transgénique, de préférence du genre Beta, ou partie de celle-ci, qui comprend une cellule végétale selon la revendication 7.

9. Plante de l'espèce Beta résistant au BNYVV ou partie de celle-ci, dans laquelle la molécule d'acide nucléique selon l'une des revendications 1 à 5 est présente de manière endogène ou dans laquelle ladite une ou lesdites plusieurs mutations, définies dans l'une des revendications 1 à 4, ont été introduites dans une molécule d'acide nucléique endogène présentant une séquence nucléotidique qui comprend la séquence selon SEQ ID NO : 1 ou qui s'hybride dans des conditions stringentes avec la séquence complémentaire à la séquence SEQ ID NO : 1, la plante ou une partie de celle-ci n'appartenant pas à la sous-espèce Beta vulgaris subsp. maritima.

10. Plante selon la revendication 8 ou 9, **caractérisée en ce que** la plante est une plante hybride ou une plante haploïde double et/ou **en ce que** la molécule d'acide nucléique ou ladite une ou lesdites plusieurs mutations se trouve(nt) sous forme hétérozygote ou homozygote.

11. Graines de la plante selon l'une des revendications 8 à 10, les graines comprenant un(e) ou plusieurs des molécules d'acide nucléique susmentionnées ou des vecteurs susmentionnés de manière transgène ou endogène.

12. Procédé de préparation d'une plante résistant au BNYVV, comprenant les étapes suivantes :
(a) mutagenèse de cellules végétales et régénération consécutive de plantes à partir des cellules végétales ayant subi la mutagenèse ou mutagenèse de plantes et
(b) identification d'une plante de (a), qui présente dans une molécule d'acide nucléique endogène ladite une ou lesdites plusieurs mutations définies dans l'une des revendications 1 à 5.

13. Procédé d'identification d'une plante du genre Beta, qui est résistante par rapport au pathogène BNYVV, **caractérisé en ce que** le procédé comprend l'étape suivante :
(i) détection de la présence d'une molécule d'acide nucléique selon la revendication 3, le polypeptide codé par celle-ci présentant (i) une glutamine (Q) dans la position qui correspond à la position 307 dans la séquence d'acides aminés de référence SEQ ID NO : 2 ou (ii) une arginine (R) dans la position qui correspond à la position 437 dans la séquence d'acides aminés de référence SEQ ID NO : 2 et/ou de l'expression de la molécule d'acide nucléique selon la revendication 4, la molécule d'acide nucléique présentant dans les sites qui correspondent aux sites dans la séquence nucléotidique de référence SEQ ID NO : 1 les substitutions nucléotidiques suivantes : (a) C au lieu de A dans la position 919 ou (b) G au lieu de A dans la position 1310 ; dans la plante ou, selon le cas, un échantillon de celle-ci ; et/ou
(ii) détection de la présence d'une molécule d'acide nucléique selon la revendication 3, le polypeptide codé par celle-ci présentant (i) une glutamine (Q) dans la position qui correspond à la position 307 dans la séquence d'acides aminés de référence SEQ ID NO: 2 et (ii) une arginine (R) dans la position qui correspond à la position 437 dans la séquence d'acides aminés de référence SEQ ID NO : 2 et/ou de l'expression de la molécule d'acide nucléique selon la revendication 4, la molécule d'acide nucléique présentant dans les sites qui correspondent aux sites dans la séquence nucléotidique de référence SEQ ID NO : 1 les substitutions nucléotidiques suivantes : (a) C au lieu de A dans la position 919 et (b) G au lieu de A dans la position 1310 ; dans la plante ou, selon le cas, un échantillon de celle-ci ; et/ou
(iii) sélection de la plante résistant au BNYVV.

14. Population de plantes comprenant des plantes selon l'une des revendications 9 ou 10 ou population de plantes qui a été identifiée et le cas échéant sélectionnée par un procédé selon la revendication 13.

15. Procédé de lutte contre une attaque par le pathogène virus des nervures jaunes nécrotiques de la betterave (BNYVV) dans la culture agricole ou horticole de plantes du genre Beta, comprenant
I) l'identification et la sélection de plantes du genre Beta à l'aide d'un procédé selon la revendication 13 et
II) culture des plantes de I) ou de leur descendants.
